(19) Europäisches Patentamt
European Patent Office
Office européen des brevets

(11) **EP 4 257 048 B1**

(12) **EUROPÄISCHE PATENTSCHRIFT**

(45) Veröffentlichungstag und Bekanntmachung des Hinweises auf die Patenterteilung:
**18.12.2024 Patentblatt 2024/51**

(21) Anmeldenummer: **22167439.3**

(22) Anmeldetag: **08.04.2022**

(51) Internationale Patentklassifikation (IPC):
*A61B 6/00* (2024.01)    *G01R 33/48* (2006.01)
*A61B 6/03* (2006.01)    *A61B 6/46* (2024.01)
*A61B 6/50* (2024.01)    *G06T 7/00* (2017.01)
*G01R 33/563* (2006.01)

(52) Gemeinsame Patentklassifikation (CPC):
**G01R 33/4812; A61B 6/032; A61B 6/4417;
A61B 6/463; A61B 6/481; A61B 6/482;
A61B 6/486; A61B 6/504; A61B 6/507;
A61B 6/5205; A61B 6/5217; A61B 6/5229;
A61B 6/5235; A61B 6/5288;** A61B 6/466;    (Forts.)

(54) **MULTIMODALE ERMITTLUNG EINER AUGMENTIERTEN SEQUENZ VON CT-PARAMETERKARTEN**

MULTIMODAL DETERMINATION OF AUGMENTED SEQUENCE OF CT PARAMETER CARDS

DÉTERMINATION MULTIMODALE D'UNE SÉQUENCE AUGMENTÉE DE CARTES DE PARAMÈTRES CT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**11.10.2023 Patentblatt 2023/41**

(73) Patentinhaber: **Siemens Healthineers AG**
**91301 Forchheim (DE)**

(72) Erfinder:
• **Lichy, Matthias**
**90489 Nürnberg (DE)**
• **Hofmann, Bernd**
**91058 Erlangen (DE)**
• **Schmidt, Bernhard**
**90766 Fürth (DE)**

(74) Vertreter: **Siemens Healthineers**
**Patent Attorneys**
**Postfach 22 16 34**
**80506 München (DE)**

(56) Entgegenhaltungen:
• **VAN BEERS BERNARD E. ET AL: "Hepatic Perfusion Parameters in Chronic Liver Disease: Dynamic CT Measurements Correlated with Disease Severity", AJR, vol. 176, March 2001 (2001-03-01), pages 667 - 687, XP055957492, Retrieved from the Internet <URL:https://www.ajronline.org/doi/epdf/10.2214/ajr.176.3.1760667> [retrieved on 20220905]**
• **GEON-HO JAHNG ET AL: "Perfusion Magnetic Resonance Imaging: A Comprehensive Update on Principles and Techniques", KOREAN JOURNAL OF RADIOLOGY, vol. 15, no. 5, 12 September 2014 (2014-09-12), pages 554, XP055199981, ISSN: 1229-6929, DOI: 10.3348/kjr.2014.15.5.554**
• **KIM SE HYUNG ET AL: "CT Perfusion of the Liver: Principles and Applications in Oncology", RADIOLOGY, vol. 272, no. 2, 24 July 2014 (2014-07-24), US, pages 322 - 344, XP055957489, ISSN: 0033-8419, DOI: 10.1148/radiol.14130091**

- **SPAHR NADINE ET AL: "Multimodal image registration for liver radioembolization planning and patient assessment", INTERNATIONAL JOURNAL OF COMPUTER ASSISTED RADIOLOGY AND SURGERY, SPRINGER, DE, vol. 14, no. 2, 22 October 2018 (2018-10-22), pages 215 - 225, XP037012487, ISSN: 1861-6410, [retrieved on 20181022], DOI: 10.1007/S11548-018-1877-5**
- **YELLESWARAPU VENKATA R ET AL: "Top-Level System Designs for Hybrid Low-Field MRI-CT with Potential of Pulmonary Imaging", SENSING AND IMAGING: AN INTERNATIONAL JOURNAL, SPRINGER US, BOSTON, vol. 15, no. 1, 11 October 2014 (2014-10-11), pages 1 - 9, XP035932311, ISSN: 1557-2064, [retrieved on 20141011], DOI: 10.1007/S11220-014-0098-4**

(52) Gemeinsame Patentklassifikation (CPC): (Forts.)
A61B 6/469; A61B 6/541; G01R 33/56366

**Beschreibung**

Die Erfindung betrifft ein Verfahren zur Ermittlung einer quantitativen Ergebnis-CT-Parameterkarte in einem Untersuchungsbereich eines Untersuchungsobjekts. Außerdem betrifft die Erfindung ein Verfahren zum Erfassen einer quantitativen Ergebnis-Parameterkarte. Weiterhin betrifft die Erfindung eine Parameterermittlungseinrichtung. Überdies betrifft die Erfindung ein multimodales medizinisches Bildgebungssystem.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können. In vielen Fällen basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems (CT-Systems) akquiriert werden.

Bei der CT-Bildgebung mit spektral aufgelösten CT-Daten, die zum Beispiel durch die Anwendung von photonenzählenden Detektoren, Dual-Source-CT-Systemen bzw. CT-Systemen mit welchen Röntgenstrahlen mit unterschiedlichen Energiespektren erzeugt werden, können unterschiedliche CT-Parameterwerte, insbesondere Konzentrationen von Kontrastmitteln (im konkreten Beispiel der CT der Jodgehalt im Gewebe) in einem Untersuchungsbereich ermittelt werden. Solche spektralen Datensätze konnten bereits ihre Eignung als Surrogate zur Bestimmung der Vitalität und der Therapieantwort im Bereich der Tumoruntersuchung und Tumortherapie beweisen.

Eine andere Form der medizinischen Bildgebung betrifft die Magnetresonanztomographie, abgekürzt mit MRT. Die Magnetresonanztomographie kann für die Erzeugung hochaufgelöster MRT-Datensätze genutzt werden, die nützliche Informationen bei einer Reihe von Erkrankungen liefern. Bei der MRT wird wie auch bei der CT der Durchfluss von Blut durch Organe, Gewebe und Blutgefäße gemessen bzw. bildlich mit Hilfe der Kontrastmittelgabe dargestellt. Eine typische Untersuchung betrifft die Leber. Hierbei werden zu unterschiedlichen Zeitpunkten die Aufnahmen wiederholt, um hierdurch Informationen über die Durchblutung und Anatomie des Organs und ggf. vorhandene Pathologien zu ermitteln.

Um den Zustand eines Tumors vergleichbar beschreiben zu können, müssen die Bildaufnahmen möglichst immer in derselben Durchblutungsphase, beispielsweise dem Zeitpunkt der maximalen Durchblutung, erfolgen.

Bei der Anwendung auf eine Darstellung der Perfusion der Leber umfasst die Multiphasen-CT normalerweise vier unterschiedliche Phasen: die Nativphase, die arterielle Phase, die parenchymatöse Phase und die venöse Phase.

In der Nativphase können Informationen über Verfettungen, aber auch Verkalkungen, Einblutungen und Reste von früheren Kontrastmitteln gewonnen werden.

Danach erreicht das Kontrastmittel über die Arteria hepatica die Leber. In der arteriellen Phase werden vor allem Perfusionsstörungen und hypervaskularisierte Tumore sichtbar.

In der umgangsprachlich als portalvenöse Phase beschriebenen Aufnahmephase flutet zusätzlich über die Vena portae Kontrastmittel in die Leber. Durch die stärkere Kontrastierung des Parenchyms können in dieser Phase auch hypovaskularisierte Tumore gut erkannt werden (bspw. Metastasen).

In der venösen Phase, auch oftmals Parenchymphase genannt, kommt es zu einer Gleichverteilung des Kontrastmittels in den verschiedenen Kompartimenten. Leberläsionen mit einer verzögerten, persistierenden Kontrastmittelaufnahme, wie zum Beispiel eine fokale noduläre Hyperplasie, können in dieser Phase sichtbar werden. In noch späteren Aufnahmen kommt es zu einer homogenen Aufnahme von Kontrastmittel, diese ist insbesondere für die Beurteilung der Ausdehnung von Nekrosen relevant.

Häufig wird aber gerade bei der Multiphasen-CT der optimale Zeitpunkt für eine Messung gerade verfehlt, was die Aussagekraft der Bildaufnahme, was den gewünschten Parameter, vorzugweise ein Parameter betreffend die maximale Kontrastmittelkonzentration, welche der maximalen Durchblutung entspricht, angeht, beeinträchtigt. Dieses Problem tritt insbesondere dann auf, wenn bei der Multiphasen-CT Bildaufnahmen nur zu wenigen Zeitpunkten, beispielsweise zu vier Zeitpunkten, korrespondierend mit den vier unterschiedlichen Phasen der Perfusion erfolgen.

Um eine möglichst breite zeitliche Abdeckung einer Organbildgebung zu erreichen, wird bei der Multiphasen-CT häufig eine nicht-spektrale Perfusions-CT-Messung mit einer höheren zeitlichen Auflösung eingesetzt. Als spektrale CT soll in diesem Zusammenhang eine CT-Bildaufnahme verstanden werden, bei der nach unterschiedlichen Spektralanteilen der detektierten Röntgenstrahlung differenziert wird. Mit einer spektralen CT-Bildaufnahme lassen sich Aussagen hinsichtlich der Quantität bzw. hinsichtlich Anteilen unterschiedlicher Materialien bzw. Stoffe in einem Untersuchungsbereich vornehmen. Beispielsweise kann eine Menge oder ortsabhängige Konzentration von Kontrastmittel, Knochen, Kalk, Fett oder Wasser ermittelt werden.

Eine nicht-spektrale CT-Bildaufnahme dagegen gibt nur Informationen über die ortsabhängige Abschwächung der Röntgenstrahlung in einem Untersuchungsbereich wieder, kann aber keine quantitativen Informationen hinsichtlich des Auftretens unterschiedlicher Materialien und deren Anteile liefern.

Mithin hat eine solche nicht-spektrale Perfusions-CT-Messung den Nachteil, dass keine Quantifizierung von Kontrastmittel, beispielsweise Iod, auf Basis der CT-Bilddaten allein erfolgen kann. Eine Parametrisierung physiologischer Daten wie Blutfluss oder Zeit zur maximalen Kontrastmittelaufnahme kann durch Hinzuziehung eines pharmakokinetischen Modells erfolgen. Im Gegensatz dazu kann bei der spektralen CT auf Basis der CT-Bilddaten allein eine Kontrastmittelmenge, insbesondere Iodmenge, ermittelt werden. Außerdem weist eine zeitlich hochaufgelöste Perfusions-CT-Messung eine vergleichsweise hohe Strahlenbelastung bzw. hierdurch bedingt eine entsprechend reduzierte Bildqualität für einzelne Zeitpunkte im Vergleich zur klassischen Multiphasen-CT mit nur wenigen Bildaufnahmen auf.

Dagegen ermöglichen neue Entwicklungen in der Magnetresonanztomographie die Aufnahme von zeitlich hochaufgelösten 3D-Datensätzen ohne Strahlenbelastung, bei denen praktisch immer auch der optimale Zeitpunkt einer maximalen Kontrastmittelmenge in einem überwachten Bereich während einer Organ-/Tumorperfusion erfasst wird. Allerdings ist ähnlich wie bei der nicht-spektralen Perfusions-CT auch bei der Magnetresonanztomographie eine Quantifizierung physiologischer Parameter nur mittels eines pharmakokinetischen Modells möglich.

Häufig kommt es vor, dass sowohl eine CT-Bildaufnahme in Form einer multiphasischen CT als auch eine MRT-Bildaufnahme von einem Patienten erfolgen und je nach therapeutischer Relevanz auch wiederholt werden.

Wie bereits erwähnt, weisen aber Aufnahmen beider Modalitäten das Problem auf, eine absolute Quantifizierung eines Kontrastmittels zu einem optimalen Zeitpunkt als Surrogat für u.a. eine Beurteilung von Tumorvitalität, insbesondere zu einem definierten Zeitpunkt zu ermitteln, bei dem eine maximale Kontrastmittelmenge in dem Untersuchungsbereich vorherrscht.

Ein Verfahren zur dynamischen kontrastmittelbasierten CT-Perfusionsmessung ist beispielsweise aus Van Beers B. E. et al., "Hepatic Perfusion Parameters in Chronic Disease: Dynamic CT Measurements Correlated with Disease Severity", AJR, 2001, vol. 176, Nr. 3, S. 667-687, bekannt. MR-Perfusionsmessungen sind beispielsweise in Jahng G.-H. et al., "Perfusion Magnetic Resonance Imaging: A Comprehensive Update on Principles and Techniques", Korean Journal of Radiology, 2014, Bd. 15, Nr. 5, S. 554-577, beschrieben.

Die genannte Aufgabe wird durch ein Verfahren zur Ermittlung einer quantitativen Ergebnis-CT-Parameterkarte in einem Untersuchungsbereich eines Untersuchungsobjekts gemäß Patentanspruch 1, ein Verfahren zum Erfassen einer quantitativen Ergebnis-CT-Parameterkarte von einem Untersuchungsbereich eines Untersuchungsobjekts gemäß Patentanspruch 11, eine Parameterermittlungseinrichtung gemäß Patentanspruch 12 und ein multimodales Bildgebungssystem gemäß Anspruch 13 gelöst.

Insbesondere können die im Zusammenhang mit den erfindungsgemäßen Verfahren beschriebenen Merkmale und Vorteile auch als entsprechende Untereinheiten der erfindungsgemäßen Parameterermittlungseinrichtung oder des erfindungsgemäßen multimodales Bildgebungssystems ausgebildet sein. Umgekehrt können die im Zusammenhang mit der erfindungsgemäßen Parameterermittlungseinrichtung oder dem erfindungsgemäßen multimodalen Bildgebungssystem beschriebenen Merkmale und Vorteile auch als entsprechende Verfahrensschritte der erfindungsgemäßen Verfahren ausgebildet sein.

Bei dem erfindungsgemäßen Verfahren zur Ermittlung einer quantitativen Ergebnis-CT-Parameterkarte in einem Untersuchungsbereich eines Untersuchungsobjekts wird eine Sequenz von quantitativen Eingangs-CT-Parameterkarten für vorbestimmte Zeitpunkte übernommen. Die vorbestimmten Zeitpunkte liegen in einem definierten bzw. vorbestimmten Zeitbereich, welcher durch die Messung definiert ist, welche einer Generierung der quantitativen Eingangs-CT-Parameterkarten zugrunde liegt. Die Messung umfasst eine spektrale Multiphasen-CT, mit der die Sequenz von quantitativen Eingangs-CT-Parameterkarten von dem Untersuchungsbereich kontrastmittelunterstützt generiert wurde. Wie bereits erwähnt, umfasst der definierte Zeitbereich auf die klinische Fragestellung vorbestimmte Zeitpunkte bzw. Messzeitpunkte, bevorzugt aber mindestens vier Messzeitpunkte, die bevorzugt die eingangs erwähnten unterschiedlichen Phasen einer Perfusionsmessung, bevorzugt einer Perfusionsmessung eines Organs, insbesondere einer Leberperfusionsmessung, abdecken. Das erfindungsgemäße Verfahren wird also bevorzugt für eine Auswertung einer Perfusionsmessung eingesetzt.

Der Ausdruck "Übernehmen" soll alle Arten der Entgegennahme von Daten umfassen, insbesondere einen Empfang über eine Empfangseinheit in einem technischen Datenübertragungssystem, welches beispielsweise eine Datenübertragungseinheit, wie zum Beispiel eine Datenübertragungsleitung, ein Sende-/Empfangssystem oder eine Datenempfangsschnittstelle umfasst. Weiterhin umfasst der Ausdruck "Übernehmen" auch einen Empfang von Daten innerhalb eines Datenverarbeitungssystems bzw. eines zur Auswertung von Messdaten, insbesondere, Rohdaten und Bilddaten, genutzten Computerprogramms. Die Übernahme der Daten erfolgt bevorzugt aus einer CT-Scaneinheit oder einer medizinischen Datenbank als Quelle.

Ein Untersuchungsbereich umfasst einen durch eine medizinische Bildgebung, insbesondere eine Multiphasen-CT und eine Magnetresonanztomographie abbildbaren Körperbereich, bevorzugt eines Organs, insbesondere einen Bereich oder Teilbereich der Leber oder des Gehirns eines Menschen. Besonders relevant sind die eingangs erwähnten Gefäße und Gewebetypen der Leber. Es soll aber darauf hingewiesen werden, dass das Verfahren auch auf andere Körperbereiche als die Leber, beispielsweise den Darm, die Milz oder das Gehirn eines Untersuchungsobjekts, insbesondere eines Menschen oder Tieres, angewendet werden kann.

Eine quantitative CT-Parameterkarte umfasst eine 2-dimensionale oder 3-dimensionale Darstellung eines ortsabhängigen Werts eines CT-Parameters zu einem vorbestimmten Zeitpunkt oder für ein vorbestimmtes Zeitintervall für einen Untersuchungsbereich. Ein CT-Parameter betrifft eine auf Basis einer CT-Bildgebung ermittelbare Messgröße.

Eine einzelne CT-Parameterkarte stellt anschaulich gesprochen eine Momentaufnahme von einem Untersuchungsbereich dar.

CT-Parameter können insbesondere sogenannte Perfusionsparameter, die einen Perfusionsprozess quantitativ kennzeichnen, umfassen. Perfusionsparameter umfassen eine Perfusion kennzeichnende Messgröße, insbesondere die ortsabhängige Kontrastmittelkonzentration oder Kontrastmittelmenge, den Blutfluss, das Blutvolumen, die mittlere Durchflusszeit oder die Gefäßpermeabilität. Die genannten Messgrößen werden vorzugsweise jeweils für unterschiedliche Gewebetypen, welche einen Tumor und gesundes Gewebe umfassen, ermittelt.

Die Ermittlung der Eingangs-CT-Parameterwerte, beispielsweise Werte, welche eine Kontrastmittelkonzentration oder Kontrastmittelmenge wiedergeben, erfolgt bevorzugt durch ein Verfahren zur Basismaterialzerlegung bzw. ein Verfahren zur Rekonstruktion bzw. Berechnung von virtuellen basismaterialgewichteten Bilddaten, insbesondere pseudo-monoenergetischen Bilddaten. Ein solches Verfahren ist aus Alvarez R.E. and Macovski A. "Energy-selective reconstructions in x-ray computed tomography", Phys. Med. Biol. 21, 733-744 (1976) bekannt.

Ein besonders geeignetes Verfahren zur Rekonstruktion bzw. Berechnung von derartig rekonstruierten Bilddaten, insbesondere pseudo-monoenergetischen Bilddaten, ist in K.L. Grant et al. "Assessment of an Advanced Image-Based Technique to Calculate Virtual Monoenergetic Computed Tomographie Images From a Dual-Energy Examination to Improve Contrast-To-Noise Ratio in Examinations Using Iodinated Contrast Media", Investigative Radiology 2014; 00: 00-00, beschrieben.

Vorteilhaft können auf Basis einer solchen Basismaterialzerlegung einzelne Anteile der durch die spektrale Multiphasen-CT erfassten Bilddaten für die Erstellung der CT-Parameterkarten, insbesondere der Eingangs-CT-Parameterkarten, genutzt werden. Auf diese Weise können neben der Abschwächung von Voxeln einer CT-Messung auch andere CT-Parameter, wie zum Beispiel eine Kontrastmittelkonzentration, ermittelt werden.

"Kontrastmittelunterstützt" soll in diesem Zusammenhang bedeuten, dass die CT-Bildaufnahme bzw. die im Folgenden noch erwähnte MRT-Bildaufnahme in Anwesenheit eines Kontrastmittels erfolgen und das Kontrastmittel durch seine Anwesenheit die Messwerte beeinflusst. Für die CT-Bildaufnahme wird bevorzugt Jod als Kontrastmittel genutzt. Dagegen werden für die MRT-Bildaufnahme bevorzugt Kontrastmittel verwendet, welche Gadolinium-Verbindungen oder Eisenoxide oder Manganverbindungen umfassen. Allgemein wird in der CT-Bildgebung ein Stoff als Kontrastmittel genutzt, der für einen bestimmten Energiewert der Röntgenstrahlung (für Jod sind es 33 keV) einen starken Anstieg der Absorption aufweist. Dagegen werden in der MR-Bildgebung Kontrastmittel eingesetzt, die einen starken Paramagnetismus aufweisen, der den benachbarten Protonen, im Wesentlichen Protonen von Wasser, ermöglicht, schneller zu relaxieren. Das Kontrastmittel erzeugt also einen starken Unterschied der Messwerte, welche in einem von dem Kontrastmittel beaufschlagten Bereich erfasst werden, im Vergleich zu Messwerten, welche in einem nicht von dem Kontrastmittel beaufschlagten Bereich erfasst werden.

"Quantitativ" soll in diesem Zusammenhang bedeuten, dass die CT-Parameterwerte auf Basis der erfassten Messwerte bezüglich ihrer absoluten Größe ermittelbar sind. Die Ermittlung quantitativer Parameterwerte ist zwar bei der spektralen CT-Messung möglich, aber nicht bei einer allein bzw. ausschließlich vorgenommenen MR-Messung.

Zudem wird eine Sequenz von kontrastmittelunterstützt generierten MRT-Bilddatensätzen übernommen. Die Sequenz umfasst eine sogenannte 4D-MRT-Bildaufnahme. 4D-MRT ist eine Abkürzung für "vierdimensionale Magnetresonanztomographie". Die 4D-MRT bildet einen zeitlich veränderlichen Untersuchungsbereich in einem definierten bzw. vorbestimmten Zeitbereich durch eine Sequenz von MRT-Bilddatensätzen, die jeweils anschaulich gesprochen jeweils eine Momentaufnahme darstellen, ab. Für diese Momentaufnahmen wird häufig auch die Bezeichnung Frame verwendet. Die Sequenz von kontrastmittelunterstützt generierten MRT-Bilddatensätzen weist im Vergleich zu der Sequenz von quantitativen Eingangs-CT-Parameterkarten eine höhere zeitliche Auflösung bis hinunter in den Sub-Sekundenbereich für den gesamten Untersuchungsbereich, vorzugsweise die gesamte Leber, auf. D.h. es wird bevorzugt ein MRT-Bilddatensatz pro Sekunde und besonders bevorzugt mehr als ein MRT-Bilddatensatz pro Sekunde generiert. Durch Anwendung von Techniken zur Bewegungskorrektur können hiermit zeitlich und räumlich hochaufgelöste Daten über einen gesamten Perfusionszyklus, bevorzugt über einen gesamten Leberperfusionszyklus, akquiriert werden. Eine Bewegungskorrektur ist insbesondere bei sich bewegenden Untersuchungsbereichen bzw. Organen, beispielsweise im Rumpfbereich eines Patienten, sinnvoll.

Eine höhere zeitliche Auflösung soll bedeuten, dass in demselben definierten Zeitbereich eine höhere Anzahl von MRT-Bilddatensätzen als CT-Parameterkarten aufgenommen werden. Bevorzugt werden in dem vordefinierten Zeitbereich 4 CT-Parameterkarten aufgenommen. Dann werden in dem definierten Zeitbereich mittels o.g. Technik bzw. bei der 4D-MRT-Bildaufnahme eine größere Anzahl von Bilddatensätzen als bei der CT aufgenommen. Je höher die Samplingrate bzw. Abtastrate gewählt wird, umso genauer können die unterschiedlichen Effekte während der Kontrastmittel An- und Abflutung dargestellt werden.

Mithin weist die Sequenz von kontrastmittelunterstützt generierten MRT-Bilddatensätzen eine höhere Anzahl von Bilddatensätzen auf als die Sequenz von quantitativen Eingangs-CT-Parameterkarten. Die Sequenz von kontrastmittelunterstützt generierten MRT-Bilddatensätzen wird vorzugsweise von einem MR-System bzw. einer MR-Scaneinheit oder von einer medizinischen Datenbank als Quelle empfangen. Die Sequenz von kontrastmittelunterstützt generierten MRT-Bilddatensätzen betrifft denselben für die Aufnahme der quantitativen Ergebnis-CT-Parameterkarte definierten Zeitbereich, d.h. der von der Sequenz von kontrastmittelunterstützt generierten MRT-Bilddatensätzen abgedeckte Zeitbereich umfasst insbesondere die mindestens zwei vorbestimmten Zeitpunkte. Da die Bildaufnahmen mit den unterschiedlichen Modalitäten in der Regel nicht gleichzeitig erfolgen können, muss der definierte Zeitraum zwischen den beiden Modalitäten abgeglichen werden. Beispielsweise wird hierzu der Start des jeweiligen Bildgebungsprozesses ermittelt und gegebenenfalls zusätzlich eine zeitliche Normierung ermittelt, um eine korrekte zeitliche Zuordnung der Bilddatensätze bzw. CT-Parameterkarten der unterschiedlichen Modalitäten zueinander vornehmen zu können. Eine korrekte zeitliche Zuordnung bedeutet, dass die beiden Datensätze (jeweils ein Bilddatensatz und eine zugeordnete CT-Parameterkarte) in derselben Phase der An- oder Abflutung des jeweiligen Kontrastmittels aufgenommen wurden. Beispielsweise kann jeweils die Kontrastmittelinjektion als Startpunkt der jeweiligen Bildgebung bzw. des definierten Zeitbereichs genutzt werden. Kommt es aufgrund geänderter Umstände zu einem unterschiedlichen Anflutungsverhalten bzw. einer unterschiedlichen Anflutungsgeschwindigkeit des jeweiligen Kontrastmittels, so muss dieses detektiert und eine zeitliche Normierung der für die jeweilige Bildgebung bzw. Erzeugung einer CT-Parameterkarte genutzten Zeitbereiche erfolgen, um eine korrekte zeitliche Zuordnung der jeweiligen Bilddatensätze bzw. CT-Parameterkarten der unterschiedlichen Messungen zueinander vornehmen zu können. Beispielsweise kann bei einem unterschiedlichen Herzminutenvolumen bei der Bildgebung mit unterschiedlichen Modalitäten eine zeitliche Anpassung bzw. Normierung durch Abgleich des Quotienten-Verhältnisses von relativen Signalintensitätswerten in zu- und abführenden Gefäßen zu einem Untersuchungsbereich, insbesondere einem Organ, vorzugsweise der Leber, mit einem Quotienten-Verhältnis der CT-Parameter bzw. CT-Parameterwerte der CT-Parameterkarten erfolgen. Einander entsprechende Zeitpunkte bzw. Phasen der Messungen unterschiedlicher Modalitäten liegen bei der Quotientenbildung dort, wo die Quotienten für unterschiedliche Modalitäten denselben Wert annehmen. Als Modalität soll ein bestimmter Typ eines Bildgebungssystems also insbesondere ein CT-System oder ein sich davon technisch grundlegend unterscheidendes MR-System verstanden werden.

Die kontrastmittelunterstützt generierten MRT-Bilddatensätze zeigen ebenfalls den Untersuchungsbereich des Untersuchungsobjekts, der durch die spektrale Multiphasen-CT erfasst wurde. Wie später noch detailliert beschrieben, repräsentieren die Sequenzen von MRT-Bilddatensätzen ortsabhängige zeitliche Signalintensitätsverläufe bzw. zeitabhängige Signalintensitätswerte in dem Untersuchungsbereich.

Weiterhin wird auf Basis der Sequenz von quantitativen Eingangs-CT-Parameterkarten und der Sequenz von kontrastmittelunterstützt generierten MRT-Bilddatensätzen eine Relation zwischen den MRT-Bilddatensätzen und den quantitativen Eingangs-CT-Parameterkarten ermittelt. Als Relation soll eine quantitative mathematische Beziehung zwischen den Signalintensitätswerten der MRT-Bilddatensätzen und den CT-Parameterwerten der quantitativen Eingangs-CT-Parameterkarten verstanden werden, mit der eine Umrechnung zwischen den unterschiedlich generierten Messdaten erfolgen kann.

Im Detail wird die Relation als eine mathematische Beziehung zwischen den zeit- und ortsabhängigen Signalintensitätswerten der den quantitativen Eingangs-CT-Parameterkarten zugeordneten MRT-Bilddatensätze und den CT-Parameterwerten dieser quantitativen Eingangs-CT-Parameterkarten an jeweils identischen Positionen bzw. einander zugeordneten Positionen bzw. einander zugeordneten Pixel- oder Voxelpositionen der MRT-Bilddatensätze bzw. CT-Parameterkarten ermittelt. Wie später noch im Detail erläutert wird, können die Positionen bzw. Pixel oder Voxel der Bilddatensätze bzw. Parameterkarten von unterschiedlichen Modalitäten einander durch eine Registrierung bzw. Bildregistrierung zugeordnet werden.

Die Relation, vorzugsweise ein funktionaler Zusammenhang, kann selbst einen oder mehrere Parameter umfassen, die auf Basis von Parameterwerten und Signalintensitätswerten von einander zeitlich und räumlich zugeordneten Voxeln der MRT-Bilddatensätze und der Eingangs-CT-Parameterkarten ermittelt werden. Die zeitliche Zuordnung wurde vorstehend ausführlich beschrieben. Wie später noch eingehend erläutert wird, kann eine räumliche Zuordnung durch eine Registrierung, insbesondere eine Bildregistrierung der zeitlich einander zugeordneten MRT-Bilddatensätze und Eingangs-CT-Parameterkarten erfolgen.

Im einfachsten Fall umfasst die Relation die Rechenvorschrift, dass die Signalintensitätswerte der MRT-Bilddatensätze, gegebenenfalls nach einer zeitlichen Normierung, durch Addition eines Offsetwerts in die (zeitlich und räumlich zugeordneten) CT-Parameterwerte umgerechnet werden.

Vorzugsweise beschreibt die Relation zumindest einen linearen Zusammenhang. Dieser Zusammenhang kann angenommen werden unter der Prämisse, dass die intravasal applizierten Kontrastmittel bei unterschiedlichen Modalitäten eine identische oder eine durch zeitliche Normierung vergleichbare Pharmakokinetik aufweisen und sich ein Signalintensitätswert eines MR-Bilddatensatzes linear zu der Konzentration eines anwesenden Kontrastmittels verhält.

Dann ist eine Änderung der gemessenen Signalintensitätswerte der MRT-Bilddatensätze proportional zu einer Änderung der bei der Multiphasen-CT im Untersuchungsbereich vorhandenen Kontrastmittelmenge zu demselben Zeitpunkt bzw. zugeordneten Zeitpunkt der unterschiedlichen Messungen der unterschiedlichen Modalitäten. Somit können quantitative Messwerte der spektralen Multiphasen-CT einer relativen Veränderung der Signalintensität in den MRT-Bilddatensätzen zugeordnet werden. Dies gilt auch dann, wenn sich die Signalintensitätswerte der MRT-Bilddatensätze zwischen zwei aufeinanderfolgenden Untersuchungen mit 4D-MRT-Bildaufnahmen bei derselben Kontrastmitteldynamik ändern. In diesem Fall ändert sich zwar die Relation, nicht aber die grundlegende Eigenschaft, dass die Änderung der gemessenen Signalintensitätswerte der MRT-Bilddatensätze proportional zu einer Änderung der bei der Multiphasen-CT im Untersuchungsbereich zu demselben bzw. dem zugeordneten Zeitpunkt vorhandenen Kontrastmittelmenge ist.

Wie später noch erläutert wird, kann dieser Umstand auch für eine Reduktion der Anzahl der eingesetzten Modalitäten bei wiederholten Untersuchungen genutzt werden.

Ein Problem bei der Vergleichbarkeit der Messungen unterschiedlicher Modalitäten könnte dann auftreten, wenn sich das Perfusionsverhalten zeitlich zwischen den unterschiedlichen Messungen unterschiedlicher Modalitäten verändert. Beispielsweise kann sich ein Gefäß verschließen, dann müsste die jeweils andere bzw. die vorhergehende Messung wiederholt werden, um ein "Matching" vornehmen zu können. Als "Matching" soll die Übertragung der Signalintensitätswerte der Sequenz von MRT-Bilddatensätzen auf die Sequenz von Eingangs-CT-Parameterkarten verstanden werden.

Auf Basis der ermittelten Relation und der MRT-Bilddatensätze wird so eine quantitative Ergebnis-CT-Parameterkarte für mindestens einen zusätzlichen Zeitpunkt, der sich von den vorbestimmten mindestens zwei Zeitpunkten, d.h. Messzeitpunkten, der spektralen Multiphasen-CT unterscheidet, ermittelt. Vorteilhaft werden die mit höherer zeitlicher Auflösung vorliegenden MRT-Bilddatensätze dazu genutzt, die zeitliche Auflösung, mit der die CT-Parameter gemessen werden, zu erhöhen. Dies wird dadurch erreicht, dass über die Ermittlung der Relation zwischen den MRT-Bilddatensätzen und den quantitativen Eingangs-CT-Parameterkarten eine Art Kalibrierung erfolgt, mit der es möglich ist, die Relativwerte der MRT-Bilddatensätze in Absolutwerte der CT-Parameter, vorzugsweise CT-Parameter einer Perfusionsmessung, ganz besonders bevorzugt CT-Parameter, welche eine Kontrastmittelkonzentration angeben, umzurechnen und so die zeitlichen Lücken der spektralen Multiphasen-CT bzw. der daraus generierten Eingangs-CT-Parameterkarten zu füllen.

Vorteilhaft werden so die Nachteile der MRT-Bilddatensätze, nämlich, dass diese nur das Änderungsverhalten von eine Perfusion kennzeichnenden Parameterwerten aber keine Absolutwerte wiedergeben, durch die Kombination mit den Absolutwerten der CT-Parameter der Multiphasen-CT kompensiert. Andererseits wird die vergleichsweise niedrige zeitliche Auflösung der Multiphasen-CT durch die Kombination mit der hoch aufgelösten Sequenz von MRT-Bilddatensätzen ebenfalls kompensiert. Vorteilhaft können aufgrund der erhöhten zeitlichen Auflösung Zeitpunkte ermittelt werden, bei denen Schwellenwerte für die CT-Parameter überschritten werden. Ein solcher Schwellenwert kann zum Beispiel eine signifikante Jodkonzentration oder einen Schwellenwert einer Jodkonzentration, welcher eine Pathologie kennzeichnet, umfassen.

Bei dem erfindungsgemäßen Verfahren zum Erfassen einer quantitativen Ergebnis-CT-Parameterkarte von einem Untersuchungsbereich eines Untersuchungsobjekts werden erste Rohdaten durch eine spektrale Multiphasen-CT von dem Untersuchungsbereich für mindestens zwei Zeitpunkte, welche in einem vorbestimmten Zeitbereich liegen, kontrastmittelunterstützt akquiriert. Auf Basis der akquirierten ersten Rohdaten wird eine Sequenz von mindestens zwei quantitativen Eingangs-CT-Parameterkarten für die mindestens zwei vorbestimmten Zeitpunkte rekonstruiert.

Zusätzlich werden zweite Rohdaten von dem Untersuchungsbereich mit im Vergleich zur Multiphasen-CT höherer zeitlicher Auflösung durch eine 4D-Magnetresonanzbildgebung in dem vorbestimmten Zeitbereich kontrastmittelunterstützt akquiriert. Auf Basis der zweiten Rohdaten wird eine Sequenz von kontrastmittelunterstützt generierten MRT-Bilddatensätzen, welche im Vergleich zu der Sequenz von quantitativen Eingangs-CT-Parameterkarten eine höhere zeitliche Auflösung aufweist, rekonstruiert.

Schließlich wird das erfindungsgemäße Verfahren zum Erfassen einer quantitativen Ergebnis-CT-Parameterkarte in einem Untersuchungsbereich eines Untersuchungsobjekts auf Basis der rekonstruierten Sequenz von quantitativen Eingangs-CT-Parameterkarten und der rekonstruierten Sequenz von MRT-Bilddatensätzen durchgeführt. Das erfindungsgemäße Verfahren zum Erfassen einer quantitativen Ergebnis-CT-Parameterkarte von einem Untersuchungsbereich eines Untersuchungsobjekts teilt die Vorteile des erfindungsgemäßen Verfahrens zur Ermittlung einer quantitativen Ergebnis-CT-Parameterkarte in einem Untersuchungsbereich eines Untersuchungsobjekts. Vorteilhaft wird durch die multimodale Messung eine Art augmentierte Sequenz von quantitativen CT-Parameterkarten, die eine erhöhte zeitliche Auflösung im Vergleich zu der Sequenz von Eingangs-CT-Parameterkarten bietet, erzeugt. Als multimodale Messung soll eine zeitlich parallele oder sequentielle Messung mit mindestens zwei unterschiedlichen Typen von medizintechnischen Bildgebungseinrichtungen, vorzugsweise einem CT-System und einem MR-System, verstanden werden. Dabei lässt sich die zeitlich sequentielle Messung technisch leichter umsetzen. Die zeitlich parallele Messung erfordert eine technisch aufwändige Integration der beiden Modalitäten. Vorteilhaft ist bei letzterer Variante, dass die Messungen unterschiedlicher Modalitäten einen identischen Zustand eines Untersuchungsobjekts bzw. Untersuchungsbereichs betreffen und so eine gute Vergleichbarkeit der Messungen der unterschiedlichen Modalitäten gegeben ist und das erfindungsgemäß durchgeführte "Matching" besonders exakt durchgeführt werden kann.

Die erfindungsgemäße Parameterermittlungseinrichtung weist eine Eingangsschnittstelle zum Übernehmen einer Sequenz von quantitativen Eingangs-CT-Parameterkarten, welche von einem Untersuchungsbereich eines Untersuchungsobjekts für mindestens zwei vorbestimmte Messzeitpunkte durch eine spektrale Multiphasen-CT kontrastmittelunterstützt generiert wurde, auf.

Die Eingangsschnittstelle der erfindungsgemäßen Parameterermittlungseinrichtung dient auch dazu, eine Sequenz von kontrastmittelunterstützt generierten MRT-Bilddatensätzen, welche im Vergleich zu der Sequenz von quantitativen Eingangs-CT-Parameterkarten eine höhere zeitliche Auflösung aufweist, zu übernehmen.

Teil der erfindungsgemäßen Parameterermittlungseinrichtung ist auch eine Relationsermittlungseinheit zum Ermitteln einer Relation zwischen den MRT-Bilddatensätzen und den quantitativen Eingangs-CT-Parameterkarten.

Die erfindungsgemäße Parameterermittlungseinrichtung umfasst zudem eine Parameterermittlungseinheit zum Ermitteln einer quantitativen Ergebnis-CT-Parameterkarte auf Basis der ermittelten Relation und der MRT-Bilddatensätze für mindestens einen zusätzlichen Zeitpunkt, der sich von den mindestens zwei vorbestimmten Zeitpunkten unterscheidet.

Vorzugsweise umfasst die erfindungsgemäße Parameterermittlungseinrichtung eine Zuordnungseinheit zum zeitlichen Zuordnen der MRT-Bilddatensätze zu den zugehörigen quantitativen Eingangs-CT-Parameterkarten für die mindestens zwei vorbestimmten Zeitpunkte. Bevorzugt umfasst die erfindungsgemäße Parameterermittlungseinrichtung auch eine Registrierungseinheit zum räumlichen Zuordnen der einander zeitlich zugeordneten quantitativen Eingangs-CT-Parameterkarten und MRT-Bilddatensätze durch eine Bildregistrierung. Eine solche Bildregistrierung kann je nach Beschaffenheit des untersuchten Objekts eine rigide Bildregistrierung oder eine elastische Bildregistrierung umfassen. Unter einer Registrierung wird verstanden, zwei Bilder oder Volumen (in diesem Fall ein MRT-Bild und eine CT-Parameterkarte) möglichst exakt aufeinander abzubilden. Dabei muss eine geometrische Transformation gefunden werden, die dies gewährleistet. Als rigide Bildregistrierung wird eine Registrierung bezeichnet, wenn sie ausschließlich Translationen und Rotationen als Bildtransformationen umfasst. Eine elastische oder deformierbare oder auch flexible Registrierung umfasst auch die Abbildung von Geraden auf Kurven.

Verfahren zur Bildregistrierung sind zum Beispiel in Nadine Spahr et al., "Multimodal image registration for liver radioembolization planning and patient assessment", International Journal of Computer Assisted Radiology and Surgery, 14, 215-225 (2019) beschrieben.

Die den mindestens zwei vorbestimmten Zeitpunkten der Multiphasen-CT zugeordneten MRT-Bilddatensätze und die diesen zugeordneten quantitativen Eingangs-CT-Parameterkarten werden von der Relationsermittlungseinheit zum Ermitteln einer Relation zwischen den MRT-Bilddatensätzen und den quantitativen Eingangs-CT-Parameterkarten genutzt. Dies ist möglich, da durch die zeitliche Zuordnung und Registrierung bereits eine eindeutige Zuordnung von Voxeln einzelner MRT-Bilddatensätze und Voxeln oder Pixeln der CT-Parameterkarten zueinander erreicht ist.

Die erfindungsgemäße Parameterermittlungseinrichtung teilt die Vorteile des erfindungsgemäßen Verfahrens zur Ermittlung einer quantitativen Ergebnis-CT-Parameterkarte in einem Untersuchungsbereich eines Untersuchungsobjekts.

Das erfindungsgemäße multimodale Bildgebungssystem weist ein CT-System zur Aufnahme einer Sequenz von kontrastmittelunterstützt generierten quantitativen Eingangs-CT-Parameterkarten von einem Untersuchungsbereich eines Untersuchungsobjekts auf.

Teil des erfindungsgemäßen multimodalen Bildgebungssystems ist auch ein MR-System zur Aufnahme einer Sequenz von kontrastmittelunterstützt generierten MRT-Bilddatensätzen von dem Untersuchungsbereich.

Das erfindungsgemäße multimodale Bildgebungssystem umfasst auch eine erfindungsgemäße Parameterermittlungseinrichtung zum Ermitteln einer Ergebnis-CT-Parameterkarte auf Basis der Sequenz von quantitativen Eingangs-CT-Parameterkarten und der Sequenz von MRT-Bilddatensätzen. Das erfindungsgemäße multimodale Bildgebungssystem teilt die Vorteile des erfindungsgemäßen Verfahrens zur Ermittlung einer quantitativen Ergebnis-CT-Parameterkarte in einem Untersuchungsbereich eines Untersuchungsobjekts.

Ein Großteil der zuvor genannten Komponenten der Parameterermittlungseinrichtung, können ganz oder teilweise in Form von Softwaremodulen in einem Prozessor eines entsprechenden Rechensystems realisiert werden, z.B. von einer Steuereinrichtung eines multimodalen medizinischen Bildgebungssystems oder einem Computer, der zur Steuerung eines solchen Systems genutzt wird. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um die Schritte des erfindungsgemäßen Verfahrens zur Ermittlung einer quantitativen Ergebnis-CT-Parameterkarte in einem Untersuchungsbereich eines Untersuchungsobjekts und die Schritte, insbesondere die Rekonstruktionsschritte des Verfahrens zum Erfassen einer quantitativen Ergebnis-CT-Parameterkarte von einem Untersuchungsbereich eines Untersuchungsobjekts auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, z.B. ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Das Rechensystem kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

Bevorzugt umfasst bei dem erfindungsgemäßen Verfahren zur Ermittlung einer quantitativen Ergebnis-CT-Parameterkarte in einem Untersuchungsbereich eines Untersuchungsobjekts der Schritt des Ermittelns der Relation einen Schritt des zeitlichen Zuordnens der MRT-Bilddatensätze zu den Eingangs-CT-Parameterkarten für die mindestens zwei vorbestimmten Zeitpunkte. Die zeitliche Zuordnung kann, wie bereits angedeutet, auf Basis des Startzeitpunkts der Kontrastmittelgabe der beiden mit unterschiedlichen Modalitäten generierten medizinischen Bildaufnahme-Sequenzen erfolgen. Weiterhin kann eine zeitliche Normierung zwischen den beiden Sequenzen erfolgen, um eine korrekte zeitliche Zuordnung zu ermöglichen. Eine solche Normierung berücksichtigt die unterschiedliche Fließgeschwindigkeit des Kontrastmittels bei den Aufnahmen mit den unterschiedlichen Modalitäten. Die Ermittlung dieser Art von zeitlicher Inkohärenz sowie eine entsprechende zeitliche Normierung kann zum Beispiel auf Basis von Messwerten an unterschiedlichen Positionen und für beide Modalitäten in dem Untersuchungsobjekt, durch eine Quotientenbildung der Messwerte für unterschiedliche Positionen sowie durch einen Vergleich der Quotienten von Bilddatensätzen und CT-Parametersätzen der unterschiedlichen Modalitäten und eine Zuordnung von Bilddatensätzen und CT-Parametersätzen mit Quotienten, die möglichst nahe beieinanderliegen bzw. deren Werte am nächsten beieinanderliegen, erfolgen.

Der Schritt des Ermittelns der Relation umfasst weiterhin bevorzugt einen Schritt des relativen räumlichen Zuordnens der zeitlich zugeordneten quantitativen Eingangs-CT-Parameterkarten und der MRT-Bilddatensätze zueinander für die mindestens zwei vorbestimmten Zeitpunkte durch eine Bildregistrierung. Als relatives räumliches Zuordnen soll verstanden werden, dass eine räumliche bzw. örtliche Zuordnung sowohl der quantitativen Eingangs-CT-Parameterkarten zu den ihnen zeitlich zugeordneten MRT-Bilddatensätzen als auch umgekehrt eine räumliche bzw. örtliche Zuordnung der MRT-Bilddatensätze zu den ihnen zeitlich zugeordneten quantitativen Eingangs-CT-Parameterkarten erfolgen kann.

Außerdem umfasst der Schritt des Ermittelns der Relation den Schritt des Ermittelns der Relation zwischen den einander zeitlich und räumlich zugeordneten MRT-Bilddatensätzen und Eingangs-CT-Parameterkarten. Wie bereits erwähnt, erfolgt die Ermittlung dieser Relation durch eine Art "Matching" der Signalintensitätswerte der MRT-Bilddatensätze und der CT-Parameterwerte der zugeordneten Eingangs-CT-Parameterkarten. Vorteilhaft erfolgt vor der Ermittlung der Relation zwischen MRT-Bilddatensätzen und Eingangs-CT-Parameterkarten durch die zeitliche Zuordnung und die Bildregistrierung eine Kompensation von zeitlichen und räumlichen Abweichungen zwischen den Bildaufnahmen bzw. Aufnahmen von Parameterkarten der unterschiedlichen Modalitäten, so dass die Genauigkeit der Ermittlung der Relation erhöht wird.

In einer Variante des erfindungsgemäßen Verfahrens zur Ermittlung einer quantitativen Ergebnis-CT-Parameterkarte in einem Untersuchungsbereich eines Untersuchungsobjekts umfasst die Ermittlung der Relation ein Verhältnis zwischen ersten CT-Parameterwerten der Eingangs-CT-Parameterkarten und Signalintensitätswerten der den Eingangs-CT-Parameterkarten zeitlich und räumlich zugeordneten MRT-Bilddatensätze.

Bildlich gesprochen erfolgt eine Art Projektion der MRT-Bilddatensätze auf die quantitativen Eingangs-CT-Parameterkarten. Vorteilhaft wird die zeitlich höher aufgelöste Sequenz von MRT-Bilddatensätzen auf die Sequenz von quantitativen Eingangs-CT-Parameterkarten projiziert bzw. es erfolgt eine Art "Matching" mit diesen, wobei die Parameterwerte der Eingangs-CT-Parameterkarten oder daraus abgeleitete Parameterwerte als Stützstellen genutzt werden, so dass die Relativinformation bzw. qualitative Information der MRT-Bilddatensätze in quantitative Information der CT-Parameter umgewandelt wird. Das "Matching" umfasst insbesondere eine zeitliche Normierung der MRT-Bilddatensätze auf die Eingangs-CT-Parameterkarten.

Besonders bevorzugt erfolgt das Ermitteln der quantitativen Ergebnis-CT-Parameterkarte zu dem mindestens einen zusätzlichen Zeitpunkt durch Extrapolation oder Interpolation auf Basis des vorbestimmten Zeitpunkts und auf Basis der ermittelten Relation. Als Extrapolation oder Interpolation soll in diesem Zusammenhang verstanden werden, dass die CT-Parameterwerte als Stützwerte und die projizierten MRT-Bilddatensätze als Extrapolationsfunktion zur Ermittlung zusätzlicher Ergebnis-CT-Parameterwerte genutzt werden.

Der absolute Wert bzw. Ergebnis-CT-Parameterwert zu dem vorbestimmten Zeitpunkt, beispielsweise ein Zeitpunkt eines Maximums eines Ergebnis-CT-Parameters, wird also durch Zuhilfenahme der CT-Parameterwerte aus den quantitativen Eingangs-CT-Parameterkarten als Stützwerte ermittelt. Vorteilhaft kann ein markanter Wert, zum Beispiel ein maximaler Wert eines CT-Parameters, besonders exakt ermittelt werden. Soll ein Wert eines Parameters ermittelt werden, der sich von dem Typ des Parameters der Eingangs-CT-Parameterkarte unterscheidet, so kann dieser auf Basis des ermittelten Ergebnis-CT-Parameterwerts und einer vorbekannten mathematischen Beziehung zwischen dem Ergebnis-CT-Parameterwert und dem sich davon unterscheidenden Parameterwert ermittelt werden.

Umfassen die Eingangs-CT-Parameter zum Beispiel sogenannte CT-Abschwächungswerte, so kann auf Basis der Kenntnis der Differenz zwischen den CT-Abschwächungswerten und den einer Kontrastmittelkonzentration zugeordneten Abschwächungswerten, die jeweils für die mindestens zwei Zeitpunkte durch eine Basismaterialzerlegung berechenbar sind, auch ein zugehöriger Wert der Kontrastmittelkonzentration, insbesondere eine Jod-konzentration für den mindestens einen zusätzlichen Zeitpunkt berechnet werden, indem diese Differenz von dem als Ergebnis-Parameterwert berechneten CT-Abschwächungswert subtrahiert wird. Vorteilhaft können also auf Basis des einmal vorgenommenen "Matchings" Parameterwerte unterschiedlichen Typs ermittelt werden.

Bevorzugt umfasst das Ermitteln der quantitativen Ergebnis-CT-Parameterkarte die Schritte des Ermittelns des mindestens einen zusätzlichen Zeitpunkts auf Basis der MRT-Bilddatensätze. Die höher aufgelöste Sequenz von MRT-Bilddatensätzen erlaubt es, markante Zeitpunkte der Untersuchung, wie zum Beispiel ein Zeitpunkt zu einem Maximum eines CT-Parameters, genauer zu erkennen bzw. zu lokalisieren.

Besonders bevorzugt umfasst der mindestens eine zusätzliche Zeitpunkt einen Zeitpunkt, zu dem auf Basis der MRT-Bilddatensätze ein maximaler CT-Parameterwert, vorzugsweise ein eine Perfusion charakterisierender CT-Parameterwert, ganz besonders bevorzugt ein maximaler Kontrastmittelkonzentrationswert, in dem Untersuchungsbereich zu erwarten ist. Vorteilhaft wird der mindestens eine zusätzliche Zeitpunkt, zu dem der maximale CT-Parameterwert auftritt, auf Basis der MRT-Bilddatensätze exakter ermittelt als nur allein auf Basis der zeitlich niedriger aufgelösten quantitativen Eingangs-CT-Parameterkarten.

Zudem erfolgt auf Basis der zusätzlichen Kenntnis der Relation zwischen den MRT-Bilddatensätzen und den quantitativen Eingangs-CT-Parameterkarten die Ermittlung des gesuchten maximalen CT-Parameterwerts, vorzugsweise ein Parameter einer CT-Perfusionsmessung, ganz besonders bevorzugt eine maximale Kontrastmittelkonzentration, exakter als allein auf Basis der quantitativen Eingangs-CT-Parameterkarten.

In einer besonders bevorzugten Variante des erfindungsgemäßen Verfahrens zur Ermittlung einer quantitativen Ergebnis-CT-Parameterkarte in einem Untersuchungsbereich eines Untersuchungsobjekts geben die Eingangs-CT-Parameterkarten und die Ergebnis-CT-Parameterkarte Parameterwerte an, die einen der folgenden CT-Parameterwerttypen umfassen:

- einen zeit- und ortsabhängigen normierten Abschwächungswert,
- einen zeit-, orts- und energieabhängigen Abschwächungswert,
- einen zeit- und ortsabhängigen Dichtewert,
- einen zeit- und ortsabhängigen Kontrastmittelkonzentrationswert,
- einen Wert, der eine zeit- und ortsabhängige Kontrastmittelmenge angibt,
- einen Wert, der eine zeit- und ortsabhängige Blutmenge angibt.

Ein normierter Abschwächungswert umfasst einen Wert, der eine ortsabhängige Röntgenabsorption normiert auf Schwächungskoeffizienten von Wasser und Luft darstellt. Häufig wird ein solcher Wert auch als "HU"-Wert bezeichnet.

Ein energieabhängiger Abschwächungswert wird bei einer spektralen CT-Bildgebung für ein bestimmtes Röntgenspektrum mit einer bestimmten Energie erfasst.

Ein Dichtewert wird auf Basis einer spektralen CT-Bildgebung und auf Basis der vorstehend erwähnten Basismaterialzerlegung durch eine Ermittlung von pseudomonochromatischen Bilddaten ermittelt.

Besonders bevorzugt wird als Dichte eine Kontrastmittelkonzentration ermittelt. Ganz besonders bevorzugt umfasst das Kontrastmittel ein jodbasiertes Kontrastmittel. Vorteilhaft können Perfusionsprozesse durch die Kontrastmittel in Körperstrukturen sichtbar gemacht werden und quantitativ ausgewertet werden. In dieser besonders bevorzugten Variante werden als quantitative Eingangs-CT-Parameterkarten, quantitative Kontrastmittelkarten bzw. Kontrastmittelkonzentrationskarten, ganz besonders bevorzugt quantitative Jodkarten bzw. Jodkonzentrationskarten verwendet. Als Ergebnis erhält man dann auf Basis der multimodalen Bildgebung Kontrastmittelkarten, insbesondere Jodkarten mit einer erhöhten zeitlichen Auflösung. Die Menge des Kontrastmittels, insbesondere bei einem Zeitpunkt einer maximalen Kontrastmittelmenge, gibt Auskunft über die ortsabhängige Durchblutung in einem Untersuchungsbereich.

Es soll an dieser Stelle nochmals ausdrücklich erwähnt werden, dass die Eingangs-CT-Parameterkarten und/oder die Ergebnis-CT-Parameterkarte bevorzugt eine Verteilung mindestens eines Perfusionsparameters wiedergeben. Wie bereits erwähnt, kann dieser Perfusionsparameter in der Regel auf Basis einer Kontrastmittelverteilung ermittelt werden. Wie bereits erwähnt, umfassen Perfusionsparameter eine Perfusion kennzeichnende Messgröße, insbesondere einer der folgenden Größen, umfassend die ortsabhängige Kontrastmittelkonzentration oder Kontrastmittelmenge, den Blutfluss, das Blutvolumen, die mittlere Durchflusszeit oder die Gefäßpermeabilität. Vorteilhaft wird durch den Gewinn an zeitlicher Auflösung im Vergleich zu einer monomodalen Herangehensweise eine exaktere Abbildung und Analyse des Perfusionsverhaltens in einem Untersuchungsbereich ermöglicht.

In einer besonders bevorzugten Variante des erfindungsgemäßen Verfahrens zur Ermittlung einer quantitativen Ergebnis-CT-Parameterkarte wird die generierte quantitative Ergebnis-CT-Parameterkarte für die Ermittlung eines pharmakokinetischen Modells genutzt. Vorteilhaft kann ein solches pharmakokinetisches Modell, welches die Dynamik eines Transports eines pharmazeutischen Stoffs in einem Untersuchungsobjekt nachbildet und beispielsweise zur Ermittlung des Zeitpunkts eines Maximums einer Kontrastmittelkonzentration in einem Untersuchungsbereich des Untersuchungsobjekts herangezogen wird, präzisiert werden.

Ganz besonders bevorzugt werden bei dem erfindungsgemäßen Verfahren zur Ermittlung einer quantitativen Ergebnis-CT-Parameterkarte auf Basis der ermittelten Relation und der MRT-Bilddatensätze eine Mehrzahl von quantitativen Ergebnis-CT-Parameterkarten erzeugt. Vorteilhaft kann die Auflösung der so entstehenden augmentierten Sequenz von quantitativen CT-Parameterkarten aufgrund der erhöhten Anzahl der gewonnenen CT-Parameterkarten weiter gesteigert werden.

Mithin kann auf Basis der ermittelten Relation und der MRT-Bilddatensätze eine zeitlich hochaufgelöste Sequenz von CT-Parameterkarten erzeugt werden. Eine zeitlich hochaufgelöste Sequenz von CT-Parameterkarten umfasst bevorzugt mindestens eine CT-Parameterkarte pro eine halbe Sekunde, besonders bevorzugt mindestens eine CT-Parameterkarte pro Sekunde.

Besonders bevorzugt erfolgt auf Basis der Kenntnis der Relation und/oder der hochaufgelösten Sequenz von CT-Parameterkarten eine monomodale Untersuchung, vorzugsweise eine Verlaufsuntersuchung. Bei dieser Art der Untersuchung wird nur eine einzige Untersuchung entweder mit einem CT-System oder einem MR-System durchgeführt und die fehlenden Informationen durch die bekannte Relation bzw. die bekannten hochaufgelösten Sequenzdaten ergänzt. Bildlich gesprochen kann also sowohl bei einer zukünftigen CT-Messung als auch bei einer zukünftigen MRT-Messung eine Abschätzung für die jeweils andere Modalität ermittelt werden, so dass eine Anwendung nur einer einzigen Modalität notwendig ist. Vorteilhaft können bei häufiger auftretenden Untersuchungen, insbesondere Verlaufsuntersuchungen, Aufwand, Zeit und Ressourcen gespart werden und die Belastung, insbesondere eine Strahlenbelastung, für den Patienten reduziert werden.

Eine Form häufig wiederkehrender Untersuchungen stellen Verlaufsuntersuchungen dar, die regelmäßig wiederkehrend einen Gesundheitszustand, beispielsweise eine Dynamik eines Tumors oder ein Ansprechen eines Tumors auf eine Therapie, dokumentieren sollen.

Besonders bevorzugt erfolgt die Ermittlung der Relation auf Basis der Prämisse, dass intravasal applizierte Kontrastmittel in der CT-Messung eine identische Pharmakokinetik zu intravasal applizierten Kontrastmitteln in der MRT-Messung aufweisen und die Änderungen der Signalintensität in der MRT-Messung einer gewissen Linearität in Abhängigkeit von der Konzentration der Kontrastmittel unterliegen, so dass die Veränderung der gemessenen Signalintensitätswerte in der MRT-Messung gleichzusetzen ist mit der Menge an Kontrastmittel in der CT-Messung zu einem der vorbestimmten Zeitpunkte. Vorteilhaft können die auf Basis der CT-Messung ermittelten Kontrastmittelkonzentrationswerte als Stützwerte genutzt werden, um weitere Kontrastmittelkonzentrationswerte, welche anderen Zeitpunkten zugeordnet sind, auf Basis der MRT-Messung zu ermitteln und so die zeitliche Auflösung einer Kontrastmittelkonzentrationsmessung zu erhöhen.

Die Erfindung wird im Folgenden unter Hinweis auf die beigefügten Figuren anhand von Ausführungsbeispielen noch einmal näher erläutert. Es zeigen:

FIG 1 ein Flussdiagramm, welches ein Verfahren zum Erfassen einer quantitativen Ergebnis-CT-Parameterkarte von einem Untersuchungsbereich eines Untersuchungsobjekts gemäß einem Ausführungsbeispiel der Erfindung veranschaulicht,

FIG 2 eine schematische Darstellung von Signalintensitätskurven an unterschiedlichen Positionen im Untersuchungsbereich für eine 4D-MRT-Bildaufnahme,

FIG 3 eine schematische Darstellung einer CT-Abschwächungswertekurve, welche auf Basis einer spektralen Multiphasen-CT-Bildaufnahme und der in FIG 2 gezeigten Signalintensitätskurve einer 4D-MRT-Bildaufnahme ermittelt wurde, sowie eines extrapolierten Jod-Konzentrationswerts für den Zeitpunkt einer maximalen Durchblutung,

FIG 4 eine schematische Darstellung eines Untersuchungsbereichs, der eine menschliche Leber umfasst,

FIG 5 eine schematische Darstellung einer Parameterermittlungseinrichtung gemäß einem Ausführungsbeispiel der Erfindung,

FIG 6 eine schematische Darstellung eines medizinischen Bildgebungssystems gemäß einem Ausführungsbeispiel der Erfindung.

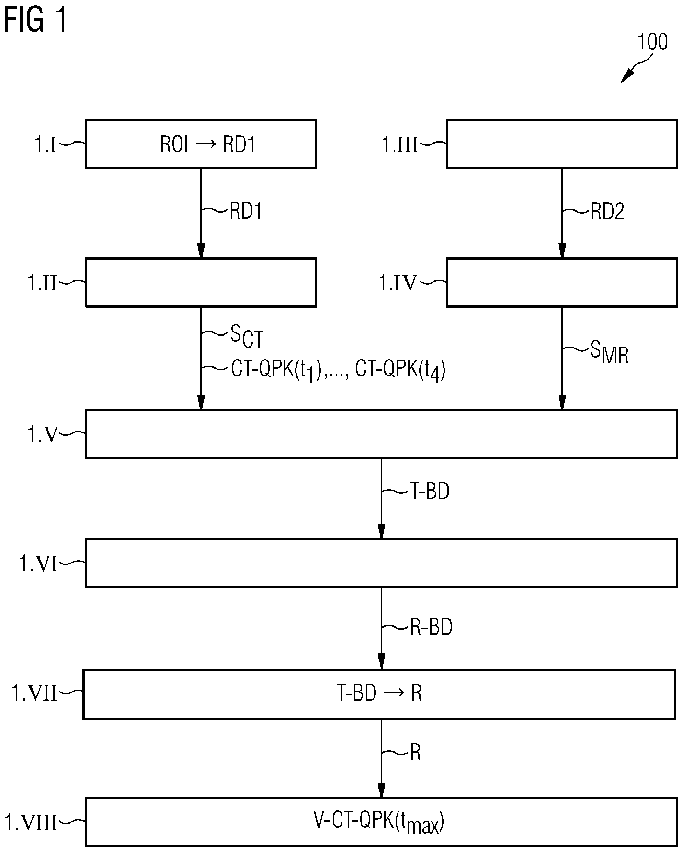

In FIG 1 ist ein Flussdiagramm 100 gezeigt, welches ein Verfahren zum Erfassen einer quantitativen Ergebnis-CT-Parameterkarte V-CT-QPK($t_{max}$) von einem Untersuchungsbereich ROI eines Untersuchungsobjekts O veranschaulicht

Bei dem Schritt 1.I werden erste Rohdaten RD1 durch eine spektrale Multiphasen-CT von dem Untersuchungsbereich ROI für vorbestimmte Zeitpunkte $t_1$, $t_2$, $t_3$, $t_4$ kontrastmittelunterstützt akquiriert. Als Kontrastmittel wird bei der spektralen Multiphasen-CT Jod genutzt. Die Erfassung der Rohdaten RD1 erfolgt spektral aufgelöst, so dass eine Konzentration eines Kontrastmittels in dem Untersuchungsbereich ROI als CT-Parameter ermittelt werden kann.

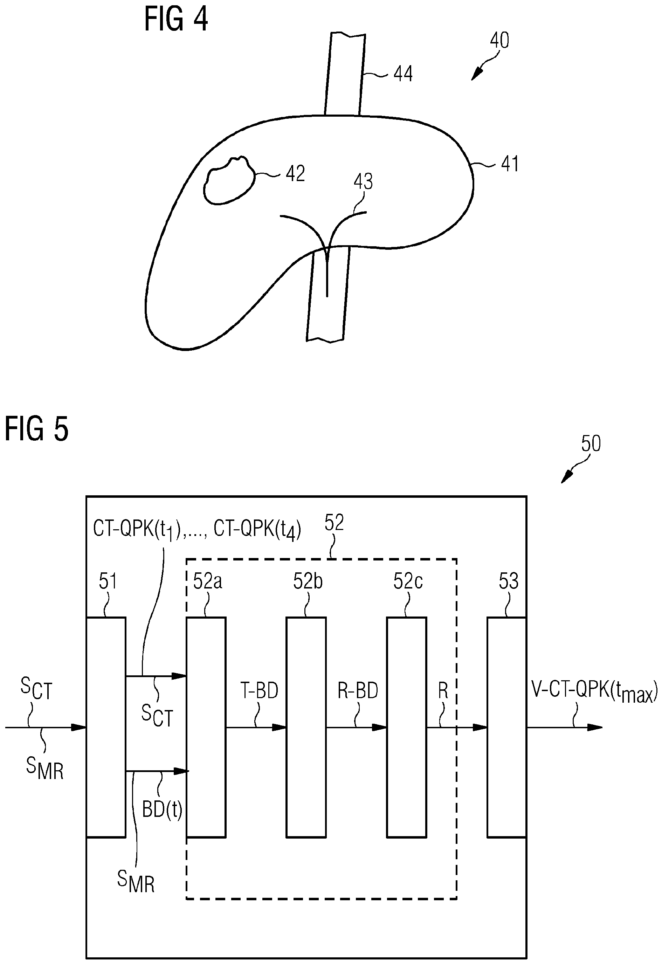

Bei dem Schritt 1.II wird eine Sequenz $S_{CT}$ von quantitativen Eingangs-CT-Parameterkarten CT-QPK($t_1$),..., CT-QPK($t_4$) auf Basis der akquirierten ersten Rohdaten RD1 für den vorbestimmten Zeitpunkten $t_1$, $t_2$, $t_3$, $t_4$ rekonstruiert. Diese Eingangs-CT-Parameterkarten CT-QPK($t_1$),..., CT-QPK($t_4$) stellen bevorzugt jeweils eine ortsabhängige Verteilung eines Kontrastmittels zu den vorbestimmten Zeitpunkten $t_1$, $t_2$, $t_3$, $t_4$ dar. Die vorbestimmten Zeitpunkte $t_1$, $t_2$, $t_3$, $t_4$ entsprechen den unterschiedlichen Phasen einer Perfusionsmessung, wie sie eingangs erläutert wurden und auch im Zusammenhang mit FIG 4 nochmals erwähnt werden.

Bei dem Schritt 1.III erfolgt ein kontrastmittelunterstütztes Akquirieren von zweiten Rohdaten RD2 von dem Untersuchungsbereich ROI mit im Vergleich zur Multiphasen-CT höherer zeitlicher Auflösung durch eine vierdimensionale Magnetresonanzbildgebung. Als Kontrastmittel wird bei der 4D-MRT-Bildgebung bevorzugt eine Gadoliniumverbindung genutzt. Alternativ werden auch Eisenoxidverbindungen oder manganhaltige Verbindungen genutzt.

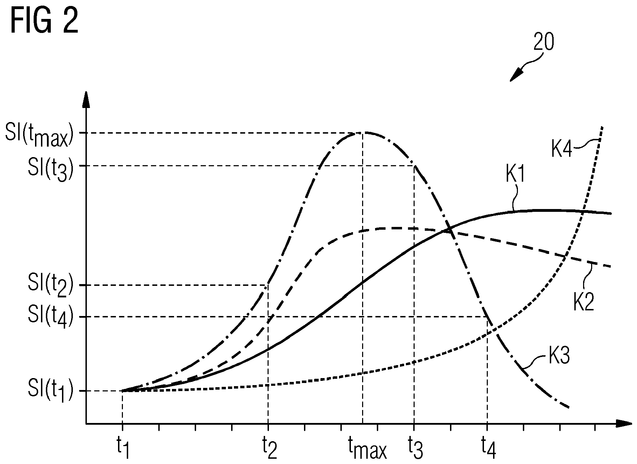

Weiterhin wird bei dem Schritt 1.IV eine Sequenz $S_{MR}$ von kontrastmittelunterstützt generierten MRT-Bilddatensätzen BD(t), welche im Vergleich zu der Sequenz $S_{CT}$ von quantitativen Eingangs-CT-Parameterkarten CT-QPK($t_1$),..., CT-QPK($t_4$) eine höhere zeitliche Auflösung aufweist, auf Basis der zweiten Rohdaten RD2 rekonstruiert. Die MRT-Bilddatensätze BD(t) zeigen jeweils ortsabhängige Signalintensitätswerte SI (siehe FIG 2) zu unterschiedlichen Zeitpunkten an.

Im Anschluss wird bei den Schritten 1.V bis 1.VIII das erfindungsgemäße Verfahren zur Ermittlung einer quantitativen Ergebnis-CT-Parameterkarte V-CT-QPK($t_{max}$) in einem Untersuchungsbereich ROI eines Untersuchungsobjekts O durchgeführt.

Bei dem Schritt 1.V erfolgt ein zeitliches Zuordnen der 4D-MRT-Bilddatensätze BD(t) zu den vorbestimmten Zeitpunkten $t_1$, $t_2$, $t_3$, $t_4$ der Multiphasen-CT, wobei den vorbestimmten Zeitpunkten $t_1$, $t_2$, $t_3$, $t_4$ zeitlich zugeordnete MRT-Bilddaten T-BD generiert werden.

Bei dem Schritt 1.VI erfolgt ein räumliches Zuordnen der einander zeitlich zugeordneten quantitativen Eingangs-CT-Parameterkarten CT-QPK ($t_1$), ..., CT-QPK($t_4$) und MRT-Bilddaten T-BD durch eine Bildregistrierung, wobei mit den quantitativen Eingangs-CT-Parameterkarten CT-QPK ($t_1$), ..., CT-QPK ($t_4$) registrierte MRT-Bilddaten R-BD generiert werden.

Bei dem Schritt 1.VII wird auf Basis der registrierten MRT-Bilddaten R-BD und der quantitativen Eingangs-CT-Parameterkarten CT-QPK ($t_1$),..., CT-QPK($t_4$) eine Relation R zwischen den zeit- und ortsabhängigen Signalintensitätswerten SI der mit den quantitativen Eingangs-CT-Parameterkarten CT-QPK($t_1$),..., CT-QPK($t_4$) registrierten MRT-Bilddaten R-BD und den durch die Registrierung den Signalintensitätswerten SI jeweils zugeordneten CT-Parameterwerten, beispielsweise Abschwächungswerten, der den registrierten MRT-Bilddaten R-BD jeweils zeitlich zugeordneten quantitativen Eingangs-CT-Parameterkarten CT-QPK($t_1$),..., CT-QPK($t_4$) ermittelt.

Bei dem Schritt 1.VIII wird eine quantitative Ergebnis-CT-Parameterkarte V-CT-QPK($t_{max}$) auf Basis der ermittelten Relation R und der Sequenz $S_{MR}$ von 4D-MRT-Bilddatensätzen BD(t) für mindestens einen zusätzlichen Zeitpunkt $t_{max}$, der sich von den vorbestimmten Zeitpunkten $t_1$, $t_2$, $t_3$, $t_4$ der Multiphasen-CT unterscheidet, ermittelt.

In FIG 2 ist eine schematische Darstellung 20 von Signalintensitätskurven K1, K2, K3, K4 an unterschiedlichen Positionen im Untersuchungsbereich für eine 4D-MRT-Bildaufnahme gezeigt. Die unterschiedlichen Signalintensitätskurven K1, K2, K3, K4 entsprechen Kontrastmittelkonzentrationen an den in FIG 4 gezeigten Positionen 41, 42, 43, 44 der Leber eines Patienten.

Eine erste Position 41 entspricht einer allgemeinen Lokalisierung der Leber, eine zweite Position 42 entspricht einer Lokalisierung eines Tumors, eine dritte Position 43 entspricht der Lokalisierung der hepatischen Arterie und eine vierte Position 44 entspricht der Lokalisation der Hohlvene.

Die erste Signalkurve K1 entspricht darüber hinaus einer Messung in der Nativphase, die zweite Signalkurve K2 entspricht einer Messung in der arteriellen Phase, die dritte Signalkurve K3 entspricht einer Messung in der portalvenösen Phase und die vierte Signalkurve K4 entspricht einer Messung in der späten bspw. venösen Phase.

Die dritte Kurve K3 wird üblicherweise für eine Beobachtung eines Tumorverhaltens herangezogen. Für die dritte Kurve werden eine Vielzahl von Momentaufnahmen zu unterschiedlichen Zeitpunkten, insbesondere zu den Zeitpunkten $t_1$, $t_2$, $t_{max}$, $t_3$ und $t_4$ aufgenommen.

Die zugehörigen Signalintensitätswerte SI($t_1$), SI($t_2$), SI($t_{max}$), SI($t_3$) und SI($t_4$) werden ebenfalls erfasst. Es soll an dieser Stelle erwähnt werden, dass die 4D-MRT-Aufnahme hochaufgelöst erfolgt und neben den genannten Zeitpunkten $t_1$, $t_2$, $t_{max}$, $t_3$ und $t_4$ MRT-Bilddaten für eine Vielzahl von zusätzlichen Zeitpunkten erfasst werden, so dass die kontinuierlich gezeichnete Kurve K3 repräsentativ ist.

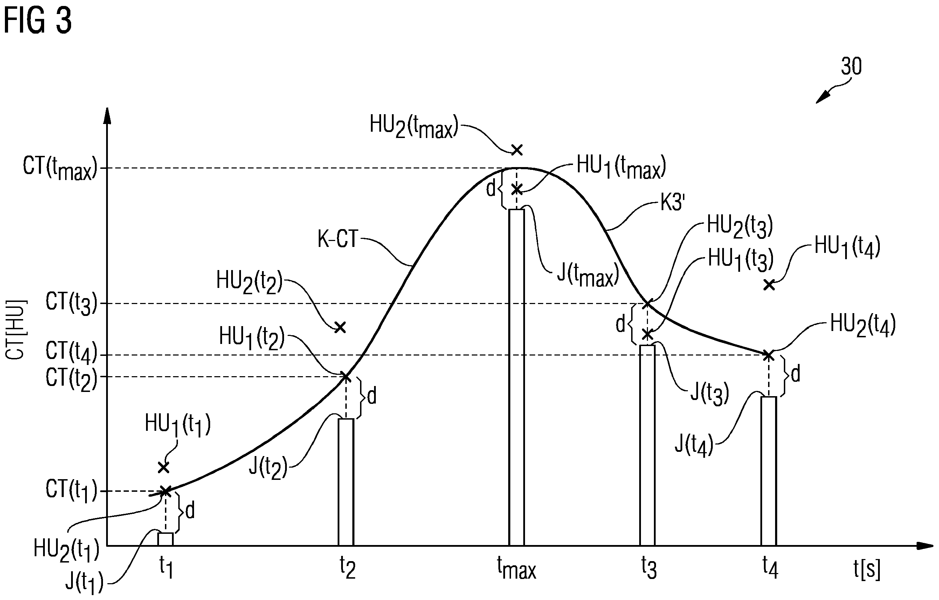

In FIG 3 ist eine schematische Darstellung 30 einer CT-Abschwächungswertekurve K-CT, welche auf Basis einer spektralen Multiphasen-CT-Bildaufnahme und der in FIG 2 gezeigten Signalintensitätskurve K3 einer 4D-MRT-Bildaufnahme ermittelt wurde, sowie eines extrapolierten Jod-Konzentrationswerts $J(t_{max})$ für den Zeitpunkt $t_{max}$ einer maximalen Durchblutung veranschaulicht.

Auf der CT-Abschwächungswertekurve K-CT liegen Abschwächungswerte $CT(t_1,)$, $CT(t_2)$, $CT(t_3)$, $CT(t_4)$, die durch die erwähnte spektrale Multiphasen-CT-Bildaufnahme ermittelt wurden. Die Abschwächungswerte $CT(t_1,)$, $CT(t_2)$, $CT(t_3)$, $CT(t_4)$ ergeben sich aus Abschwächungswerten einer spektralen Messung bzw. einer Dual-Energy-Messung für eine erste Energie $E_1$: $HU_1(t_1,)$, $HU_1(t_2)$, $HU_1(t_3)$, $HU_1(t_4)$ und eine zweite Energie $E_2$: $HU_2(t_1)$, $HU_2(t_2)$, $HU_2(t_3)$, $HU_2(t_4)$. Aus den Werten der spektralen Messung lassen sich auch einer Jodkonzentration bzw. Jodmenge zugeordnete Abschwächungswerte $J(t_1,)$, $J(t_2)$, $J(t3)$, $J(t_4)$ durch die bereits mehrfach erwähnte Methode der Basismaterialzerlegung und eine Ermittlung von pseudo-monoenergetischen Bilddaten berechnen. Die auf Basis der spektralen Multiphasen-CT sowie der 4D-MRT-Aufnahme ermittelte Kurve K-CT von Abschwächungswerten wurde durch ein "Matching" zwischen den Abschwächungswerten $CT(t_1,)$, $CT(t_2)$, $CT(t_3)$, $CT(t_4)$ der spektralen Multiphasen-CT und der in FIG 2 gezeigten Kurve K3 einer 4D-MRT-Aufnahme ermittelt.

Anschaulich gesprochen wird die Signalintensitätskurve K3 an die Abschwächungswerte $CT(t_1,)$, $CT(t_2)$, $CT(t_3)$, $CT(t_4)$ zu den vier Zeitpunkten $t_1$, $t_2$, $t_3$ und $t_4$, für die auch jeweils eine Jod-Konzentration durch die spektrale Multiphasen-CT-Aufnahme ermittelt wurde, angepasst.

Die Anpassung kann zum Beispiel durch die folgende Rechenvorschrift

$$CT(t_k) = a*SI(t_k) + b, \quad (k = 1, ..., 4)$$

und

eine Ermittlung der Parameter a und b unter Anwendung der Methode der kleinsten Quadrate erfolgen. Im besten Fall weist der Parameter a den Wert 1 auf und es erfolgt bildlich gesprochen nur eine Verschiebung der Kurve K3 in Richtung der Ordinate.

Anhand der ermittelten Parameter kann dann eine Kurve $K-CT(t_k)$ von Abschwächungswerten ermittelt werden, wobei k nun zusätzlich zu den Werten 1 bis 4 noch weitere Werte annehmen kann bzw. die Auflösung der Kurve K-CT nun höher ist als nur den vier durch die spektrale CT-Messung erfassten Messwerten entsprechend.

Auf Basis dieser Kurve $K-CT(t_k)$ kann nun auch, wie in FIG 3 gezeigt, ein Maximalwert $CT(t_{max})$ der Abschwächung, bei dem auch eine maximale Kontrastmittelkonzentration in der hepatischen Arterie zu erwarten ist, abgelesen werden.

Weiterhin kann auf Basis der Kenntnis der Differenz d zwischen den CT-Abschwächungswerten $CT(t_1,)$, $CT(t_2)$, $CT(t_3)$, $CT(t_4)$ und den einer Kontrastmittelkonzentration zugeordneten Abschwächungswerten $J(t_1)$, $J(t_2)$, $J(t_3)$, $J(t_4)$, die für die vier Zeitpunkte $t_1$, $t_2$, $t_3$ und $t_4$ durch eine Basismaterialzerlegung berechenbar sind, auch ein zugehöriger Wert der Kontrastmittelkonzentration, insbesondere eine Jodkonzentration $J(t_{max})$ berechnet werden. Dabei wird die Differenz d einfach von dem CT-Abschwächungswert $CT(t_{max})$ zu dem Zeitpunkt $t_{max}$ einer maximalen Durchblutung abgezogen.

Somit kann aus der angepassten Kurve $K-CT(t_k)$ der Zeitpunkt $t_{max}$ der maximalen Kontrastmittelkonzentration abgelesen werden und es können Werte der Kontrastmittelkonzentration und insbesondere ein maximaler Kontrastmittelkonzentrationswert $J(t_{max})$ bzw. eine maximale Kontrastmittelmenge in der hepatischen Arterie berechnet werden.

In FIG 4 ist eine schematische Darstellung 40 eines Untersuchungsbereichs, der eine menschliche Leber 41 umfasst, gezeigt.

Die menschliche Leber 41 umfasst in der in FIG 4 gezeigten Darstellung einen Tumor 42, der oben links in der Leber 41 gezeigt ist. Weiterhin wird die Leber 41 von der hepatischen Arterie 43 mit frischem Blut gespeist. Die hepatische Arterie 43 ist im Zentrum der Leber 41 dargestellt. Im Hintergrund, d.h. teilweise von der Leber 41 verdeckt und in FIG 4 vertikal verlaufend ist die Hohlvene 44 dargestellt.

In FIG 5 ist eine schematische Darstellung einer Parameterermittlungseinrichtung 50 gemäß einem Ausführungsbeispiel der Erfindung veranschaulicht.

Die Parameterermittlungseinrichtung 50 umfasst eine Eingangsschnittstelle 51. Die Eingangsschnittstelle 51 ist dazu eingerichtet, eine Sequenz $S_{CT}$ von quantitativen Eingangs-CT-Parameterkarten CT-QPK $(t_1)$, ..., CT-QPK $(t_4)$ von einem Untersuchungsbereich ROI eines Untersuchungsobjekts O, welche durch eine spektrale Multiphasen-CT kontrastmittelunterstützt für vorbestimmte Zeitpunkte $t_1$, $t_2$, $t_3$, $t_4$ generiert wurden, zu empfangen.

Außerdem ist die Eingangsschnittstelle 51 dazu eingerichtet, eine Sequenz $S_{MR}$ von kontrastmittelunterstützt generierten MRT-Bilddatensätzen BD(t), welche im Vergleich zu der Sequenz $S_{CT}$ von quantitativen Eingangs-CT-Parameterkarten CT-QPK$(t_1)$, ..., CT-QPK$(t_4)$ eine höhere zeitliche Auflösung aufweist, zu empfangen.

Teil der Parameterermittlungseinrichtung 50 ist auch eine Relationsermittlungseinheit 52. Die Relationsermittlungseinheit 52 ist dazu eingerichtet ein "Matching" zwischen den durch unterschiedliche Modalitäten erfassten Sequenzen $S_{MR}$, $S_{CT}$ durchzuführen.

Hierzu umfasst die Relationsermittlungseinheit 52 eine Zuordnungseinheit 52a zum zeitlichen Zuordnen der MRT-Bilddatensätze BD(t) zu den vorbestimmten Zeitpunkten $t_1$, $t_2$, $t_3$, $t_4$ der Multiphasen-CT, wobei den vorbestimmten Zeitpunkten $t_1$, $t_2$, $t_3$, $t_4$ zeitlich zugeordnete MRT-Bilddaten T-BD generiert werden.

Die Relationsermittlungseinheit 52 umfasst auch eine Registrierungseinheit 52b zum räumlichen Zuordnen der rekonstruierten quantitativen Eingangs-CT-Parameterkarten CT-QPK($t_1$), ..., CT-QPK($t_4$) und der rekonstruierten MRT-Bilddatensätze BD(t) durch eine Bildregistrierung, wobei mit den quantitativen Eingangs-CT-Parameterkarten CT-QPK ($t_1$), ..., CT-QPK($t_4$) registrierte MRT-Bilddaten R-BD generiert werden.

Die Relationsermittlungseinheit 52 umfasst zudem eine Relationsermittlungs-Untereinheit 52c zum Ermitteln einer Relation R zwischen den Signalintensitätswerten der registrierten MRT-Bilddaten R-BD und den CT-Parameterwerten der zugeordneten quantitativen Eingangs-CT-Parameterkarten CT-QPK($t_1$), ..., CT-QPK($t_4$). Die Relation R wird zwischen den zeit- und ortsabhängigen Signalintensitätswerten SI der mit den quantitativen Eingangs-CT-Parameterkarten CT-QPK ($t_1$), ..., CT-QPK ($t_4$) registrierten MRT-Bilddaten R-BD und den CT-Parameterwerten von durch die Registrierung Pixeln oder Voxeln der MRT-Bilddaten räumlich zugeordneten Pixeln oder Voxeln der den registrierten MRT-Bilddaten R-BD jeweils durch die zeitliche Zuordnung zugeordneten Eingangs-CT-Parameterkarten CT-QPK($t_1$),..., CT-QPK($t_4$) ermittelt.

Überdies umfasst die Parameterermittlungseinrichtung 50 eine Parameterermittlungseinheit 53 zum Ermitteln einer quantitativen Ergebnis-Parameterkarte V-CT-QPK($t_{max}$) auf Basis der ermittelten Relation R und der rekonstruierten Bilddatensätze BD(t) für mindestens einen zusätzlichen Zeitpunkt $t_{max}$.

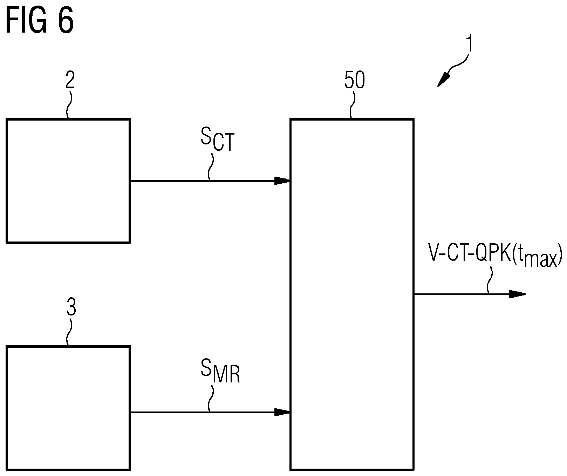

In FIG 6 ist eine schematische Darstellung eines medizinischen Bildgebungssystems 1 gemäß einem Ausführungsbeispiel der Erfindung veranschaulicht. Das medizinische Bildgebungssystem 1 umfasst ein CT-System 2, ein MR-System 3 und die in FIG 5 dargestellte Parameterermittlungseinrichtung 50. Die Parameterermittlungseinrichtung 50 empfängt von dem CT-System eine Sequenz $S_{CT}$ von kontrastmittelunterstützt generierten quantitativen Eingangs-CT-Parameterkarten CT-QPK($t_1$), ..., CT-QPK($t_4$) und von dem MR-System 3 eine Sequenz $S_{MR}$ von kontrastmittelunterstützt generierten MRT-Bilddatensätzen BD(t) und erzeugt, wie im Zusammenhang mit FIG 1 bis FIG 5 ausführlich erläutert, eine Ergebnis-CT-Parameterkarte V-CT-QPK($t_{max}$) für einen zusätzlichen Zeitpunkt $t_{max}$.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zur Ermittlung einer quantitativen Ergebnis-CT-Parameterkarte (V-CT-QPK($t_{max}$)) in einem Untersuchungsbereich (ROI) eines Untersuchungsobjekts (O), aufweisend die Schritte:

   - Übernehmen einer Sequenz ($S_{CT}$) von quantitativen Eingangs-CT-Parameterkarten (CT-QPK($t_1$), CT-QPK($t_2$)) für mindestens zwei vorbestimmte Zeitpunkte ($t_1$, $t_2$, $t_3$, $t_4$), wobei die Sequenz ($S_{CT}$) durch eine spektrale Multiphasen-CT von dem Untersuchungsbereich (ROI) kontrastmittelunterstützt generiert wurde,
   - Empfangen einer Sequenz ($S_{MR}$) von kontrastmittelunterstützt generierten MRT-Bilddatensätzen (BD(t)), welche im Vergleich zu der Sequenz ($S_{CT}$) von Eingangs-CT-Parameterkarten (CT-QPK($t_1$), CT-QPK($t_2$), CT-QPK($t_3$), CT-QPK($t_4$)) eine höhere zeitliche Auflösung aufweist,
   - Ermitteln einer Relation (R) zwischen den MRT-Bilddatensätzen (BD(t)) und den Eingangs-CT-Parameterkarten (CT-QPK($t_1$), CT-QPK($t_2$), CT-QPK($t_3$), CT-QPK($t_4$)),
   - Ermitteln der Ergebnis-CT-Parameterkarte (V-CT-QPK($t_{max}$)) auf Basis der ermittelten Relation (R) und der MRT-Bilddatensätze (BD(t)) für mindestens einen zusätzlichen Zeitpunkt ($t_{max}$), welcher sich von den vorbestimmten Zeitpunkten ($t_1$, $t_2$, $t_3$, $t_4$) unterscheidet.

2. Verfahren nach Anspruch 1, wobei das Ermitteln der Relation (R) die Schritte umfasst:

   - zeitliches Zuordnen der MRT-Bilddatensätze (BD(t)) zu den Eingangs-CT-Parameterkarten (CT-QPK($t_1$), CT-QPK($t_2$), CT-QPK($t_3$), CT-QPK($t_4$)) für die mindestens zwei vorbestimmten Zeitpunkte ($t_1$, $t_2$, $t_3$, $t_4$),
   - relatives räumliches Zuordnen der quantitativen Eingangs-CT-Parameterkarten (CT-QPK($t_1$), CT-QPK($t_2$)) und der MRT-Bilddatensätze (BD(t)) durch eine Bildregistrierung für die mindestens zwei vorbestimmten Zeitpunkte ($t_1$, $t_2$, $t_3$, $t_4$),
   - Ermitteln der Relation (R) zwischen den einander zeitlich und räumlich zugeordneten 4D-MRT-Bilddatensätzen (BD(t)) und Eingangs-CT-Parameterkarten (CT-QPK($t_1$), CT-QPK($t_2$), CT-QPK($t_3$), CT-QPK ($t_4$)) .

3. Verfahren nach Anspruch 2, wobei die Ermittlung der Relation (R) ein Verhältnis zwischen ersten CT-Parameterwerten (CT($t_1$), CT($t_2$), CT($t_3$), CT($t_4$)) der Eingangs-CT-Parameterkarten (CT-QPK($t_1$), CT-QPK($t_2$), CT-QPK($t_3$), CT-QPK($t_4$)) und Signalintensitätswerten (SI($t_1$), SI($t_2$), SI($t_3$), SI($t_4$)) der den Eingangs-CT-Parameterkarten (CT-QPK($t_1$), CT-QPK($t_2$), CT-QPK($t_3$), CT-QPK($t_4$)) zeitlich und räumlich zugeordneten MRT-Bilddatensätze (BD(t)) umfasst.

4. Verfahren nach Anspruch 3, wobei das Ermitteln der Ergebnis-CT-Parameterkarte (V-CT-QPK($t_{max}$)) zu dem mindestens einen zusätzlichen Zeitpunkt ($t_{max}$) durch Extrapolation oder Interpolation auf Basis der ersten CT-Parameterwerte (CT($t_1$), CT($t_2$), CT($t_3$), CT($t_4$)) der Eingangs-CT-Parameterkarten (CT-QPK($t_1$), CT-QPK($t_2$), CT-QPK($t_3$), CT-QPK($t_4$)) und auf Basis der ermittelten Relation (R) erfolgt.

5. Verfahren nach einem der vorstehenden Ansprüche, wobei der mindestens eine zusätzliche Zeitpunkt ($t_{max}$) auf Basis der MRT-Bilddatensätze (BD(t)) ermittelt wird.

6. Verfahren nach einem der vorstehenden Ansprüche, wobei der mindestens eine zusätzliche Zeitpunkt ($t_{max}$) einen Zeitpunkt ($t_{max}$) umfasst, zu dem auf Basis der MRT-Bilddatensätze (BD(t)) ein maximaler CT-Parameterwert, vorzugsweise ein maximaler Kontrastmittelkonzentrationswert (J($t_{max}$)), in dem Untersuchungsbereich (ROI) zu erwarten ist.

7. Verfahren nach einem der vorstehenden Ansprüche, wobei die Eingangs-CT-Parameterkarten (CT-QPK($t_1$), CT-QPK($t_2$), CT-QPK($t_3$), CT-QPK($t_4$)) und/oder die Ergebnis-CT-Parameterkarte (V-CT-QPK($t_{max}$)) einen Parameterwert mit mindestens einem der folgenden CT-Parameterwerttypen angeben:

    - einen normierten Abschwächungswert (CT($t_1$), CT($t_2$), CT($t_3$) CT($t_4$), CT($t_{max}$)),
    - einen energieabhängigen Abschwächungswert (HU($t_1$), HU($t_2$), HU($t_3$), HU($t_4$), HU($t_{max}$)),
    - einen Dichtewert,
    - einen Kontrastmittelkonzentrationswert (J($t_1$), J($t_2$), J($t_3$), J($t_4$), J($t_{max}$)) .

8. Verfahren nach einem der vorstehenden Ansprüche, wobei die Eingangs-CT-Parameterkarten (CT-QPK($t_1$), CT-QPK($t_2$), CT-QPK($t_3$), CT-QPK($t_4$)) und/oder die Ergebnis-CT-Parameterkarte (V-CT-QPK($t_{max}$)) eine Verteilung mindestens eines Perfusionsparameters wiedergeben.

9. Verfahren nach einem der vorstehenden Ansprüche, wobei auf Basis der ermittelten Relation (R) und der MRT-Bilddatensätze (BD(t)) eine zeitlich hochaufgelöste Sequenz von CT-Parameterkarten (CT-QPK($t_1$), CT-QPK($t_2$), CT-QPK($t_3$), CT-QPK($t_4$), V-CT-QPK($t_{max}$)) erzeugt wird.

10. Verfahren nach Anspruch 9, wobei auf Basis der zeitlich hochaufgelösten Sequenz von CT-Parameterkarten (CT-QPK($t_1$), CT-QPK($t_2$), CT-QPK($t_3$), CT-QPK($t_4$), V-CT-QPK($t_{max}$)) und/oder der Relation (R) eine monomodale Untersuchung, vorzugsweise eine Verlaufsuntersuchung, erfolgt.

11. Verfahren zum Erfassen einer quantitativen Ergebnis-CT-Parameterkarte (V-CT-QPK($t_{max}$)) von einem Untersuchungsbereich (ROI) eines Untersuchungsobjekts (O), aufweisend die Schritte:

    - kontrastmittelunterstütztes Akquirieren von ersten Rohdaten (RD1) durch eine spektrale Multiphasen-CT von dem Untersuchungsbereich (ROI) für mindestens zwei vorbestimmte Zeitpunkte ($t_1$, $t_2$, $t_3$, $t_4$),
    - Rekonstruieren einer Sequenz ($S_{CT}$) von quantitativen Eingangs-CT-Parameterkarten (CT-QPK($t_1$), CT-QPK($t_2$), CT-QPK($t_3$), CT-QPK($t_4$)) auf Basis der akquirierten ersten Rohdaten (RD1) für die mindestens zwei vorbestimmten Zeitpunkte ($t_1$, $t_2$, $t_3$, $t_4$),
    - kontrastmittelunterstütztes Akquirieren von zweiten Rohdaten (RD2) von dem Untersuchungsbereich (ROI) mit im Vergleich zur Multiphasen-CT höherer zeitlicher Auflösung durch eine 4D-MRT-Messung,
    - Rekonstruieren einer Sequenz ($S_{MR}$) von kontrastmittelunterstützt generierten MRT-Bilddatensätzen (BD(t)), welche im Vergleich zu der Sequenz ($S_{CT}$) von Eingangs-CT-Parameterkarten (CT-QPK($t_1$), CT-QPK($t_2$), CT-QPK($t_3$), CT-QPK($t_4$)) eine höhere zeitliche Auflösung aufweist, auf Basis der zweiten Rohdaten (RD2),
    - Durchführen des Verfahrens nach einem der Ansprüche 1 bis 10 auf Basis der Sequenz ($S_{CT}$) von Eingangs-CT-Parameterkarten (CT-QPK($t_1$), CT-QPK($t_2$), CT-QPK($t_3$), CT-QPK($t_4$)) und der Sequenz ($S_{MR}$) von kontrastmittelunterstützt generierten MRT-Bilddatensätzen (BD(t)).

12. Parameterermittlungseinrichtung (50), aufweisend:

- eine Eingangsschnittstelle (51)

- zum Übernehmen einer Sequenz ($S_{CT}$) von quantitativen Eingangs-CT-Parameterkarten (CT-QPK($t_1$), CT-QPK($t_2$), CT-QPK($t_3$), CT-QPK($t_4$)) von einem Untersuchungsbereich (ROI) eines Untersuchungsobjekts (O) für mindestens zwei vorbestimmte Zeitpunkte ($t_1$, $t_2$, $t_3$, $t_4$), wobei die Sequenz ($S_{CT}$) durch eine spektrale Multiphasen-CT kontrastmittelunterstützt generiert wurde,
- und zum Übernehmen einer Sequenz ($S_{MR}$) von kontrastmittelunterstützt generierten MRT-Bilddatensätzen (BD(t)), welche im Vergleich zu der Sequenz ($S_{CT}$) von Eingangs-CT-Parameterkarten (CT-QPK($t_1$), CT-QPK($t_2$), CT-QPK($t_3$), CT-QPK($t_4$)) eine höhere zeitliche Auflösung aufweist,

- eine Relationsermittlungseinheit (52) zum Ermitteln einer Relation (R) zwischen den MRT-Bilddatensätzen (BD(t)) und den Eingangs-CT-Parameterkarten (CT-QPK($t_1$), CT-QPK($t_2$), CT-QPK($t_3$), CT-QPK($t_4$)),
- eine Parameterermittlungseinheit (53) zum Ermitteln einer Ergebnis-CT-Parameterkarte (V-CT-QPK($t_{max}$)) auf Basis der ermittelten Relation (R) und der MRT-Bilddatensätze (BD(t)) für mindestens einen zusätzlichen Zeitpunkt ($t_{max}$), welcher sich von den vorbestimmten Zeitpunkten ($t_1$, $t_2$, $t_3$, $t_4$) unterscheidet.

13. Multimodales medizinisches Bildgebungssystem (1), aufweisend:

- ein CT-System (2) zur Aufnahme einer Sequenz ($S_{CT}$) von quantitativen Eingangs-CT-Parameterkarten (CT-QPK($t_1$), CT-QPK($t_2$), CT-QPK($t_3$), CT-QPK($t_4$)) von einem Untersuchungsbereich (ROI) eines Untersuchungsobjekts (O),
- ein MR-System (3) zur Aufnahme einer Sequenz ($S_{MR}$) von kontrastmittelunterstützt generierten MRT-Bilddatensätzen (BD(t)) von dem Untersuchungsbereich (ROI),
- eine Parameterermittlungseinrichtung (50) nach Anspruch 12 zum Ermitteln einer Ergebnis-CT-Parameterkarte (V-CT-QPK($t_{max}$)) auf Basis der Sequenz ($S_{CT}$) von quantitativen Eingangs-CT-Parameterkarten (CT-QPK($t_1$), CT-QPK($t_2$), CT-QPK($t_3$), CT-QPK($t_4$)) und der Sequenz ($S_{MR}$) von kontrastmittelunterstützt generierten MRT-Bilddatensätzen (BD(t)).

14. Computerprogrammprodukt, umfassend Befehle, die bei der Ausführung des Programms durch einen Computer diesen veranlassen, die Schritte des Verfahrens nach einem der Ansprüche 1 bis 11 auszuführen.

15. Computerlesbares Speichermedium, umfassend Befehle, die bei der Ausführung durch einen Computer diesen veranlassen, die Schritte des Verfahrens nach einem der Ansprüche 1 bis 11 auszuführen.

**Claims**

1. Method for determining a quantitative result CT parameter map (V-CT-QPK($t_{max}$)) in a region of interest (ROI) of an object under examination (O), having the following steps:

- acquiring a sequence ($S_{CT}$) of quantitative input CT parameter maps (CT-QPK($t_1$), CT-QPK($t_2$)) for at least two predetermined points in time ($t_1$, $t_2$, $t_3$, $t_4$), wherein the sequence ($S_{CT}$) was generated in a contrast-enhanced manner by spectral multiphase CT of the region of interest (ROI),
- receiving a sequence ($S_{MR}$) of MRI datasets (BD(t)) generated in a contrast-enhanced manner which has a higher temporal resolution compared to the sequence ($S_{CT}$) of input CT parameter maps (CT-QPK($t_1$), CT-QPK($t_2$), CT-QPK($t_3$), CT-QPK($t_4$)),
- determining a relation (R) between the MRI image datasets (BD(t)) and the input CT parameter maps (CT-QPK($t_1$), CT-QPK($t_2$), CT-QPK($t_3$), CT-QPK($t_4$)),
- determining the result CT parameter map (V-CT-QPK($t_{max}$)) based on the determined relation (R) and the MRI image datasets (BD(t)) for at least one additional point in time ($t_{max}$) which is different from the predetermined points in time ($t_1$, $t_2$, $t_3$, $t_4$).

2. Method according to claim 1, wherein determining the relation (R) comprises the following steps:

- temporally assigning the MRI image datasets (BD(t)) to the input CT parameter maps (CT-QPK($t_1$), CT-QPK($t_2$), CT-QPK($t_3$), CT-QPK($t_4$)) for the at least two predetermined points in time ($t_1$, $t_2$, $t_3$, $t_4$),

- relative spatial assignment of the quantitative input CT parameter maps (CT-QPK($t_1$), CT-QPK($t_2$)) and the MRI image datasets (BD(t)) by means of image registration for the at least two predetermined points in time ($t_1$, $t_2$, $t_3$, $t_4$),
- determining the relation (R) between the 4D MRI image datasets (BD(t)) and input CT parameter maps (CT-QPK($t_1$), CT-QPK($t_2$), CT-QPK($t_3$), CT-QPK($t_4$)) temporally and spatially assigned to each other.

3. Method according to claim 2, wherein determining the relation (R) involves a ratio between first CT parameter values (CT($t_1$), CT($t_2$), CT($t_3$), CT($t_4$)) of the input CT parameter maps (CT-QPK($t_1$), CT-QPK($t_2$), CT-QPK($t_3$), CT-QPK($t_4$)) and signal intensity values (SI($t_1$), SI($t_2$), SI($t_3$), SI($t_4$)) of the MRI image datasets (BD(t)) temporally and spatially assigned to the input CT parameter maps (CT-QPK($t_1$), CT-QPK($t_2$), CT-QPK($t_3$), CT-QPK($t_4$)).

4. Method according to claim 3, wherein the result CT parameter map (V-CT-QPK($t_{max}$)) at the at least one additional point in time ($t_{max}$) is determined by extrapolation or interpolation on the basis of the first CT parameter values (CT($t_1$), CT($t_2$), CT($t_3$), CT($t_4$)) of the input CT parameter maps (CT-QPK($t_1$), CT-QPK($t_2$), CT-QPK($t_3$), CT-QPK($t_4$)) and on the basis of the determined relation (R).

5. Method according to one of the preceding claims, wherein the at least one additional point in time ($t_{max}$) is determined on the basis of the MRI image datasets (BD(t)).

6. Method according to one of the preceding claims, wherein the at least one additional point in time ($t_{max}$) includes a point in time ($t_{max}$) at which, on the basis of the MRI image datasets (BD(t)), a maximum CT parameter value, preferably a maximum contrast agent concentration value (J($t_{max}$)), can be expected in the region of interest (ROI).

7. Method according to one of the preceding claims, wherein the input CT parameter maps (CT-QPK($t_1$), CT-QPK($t_2$), CT-QPK($t_3$), CT-QPK($t_4$)) and/or the result CT parameter map (V-CT-QPK($t_{max}$)) indicate a parameter value of at least one of the following CT parameter value types:

   - a normalised attenuation value (CT($t_1$), CT($t_2$), CT($t_3$) CT($t_4$), CT($t_{max}$)),
   - an energy-dependent attenuation value (HU($t_1$), HU($t_2$), HU($t_3$), HU($t_4$), HU($t_{max}$)),
   - a density value,
   - a contrast agent concentration value (J($t_2$), J($t_2$), J($t_3$), J($t_4$) , J($t_{max}$)) .

8. Method according to one of the preceding claims, wherein the input CT parameter maps (CT-QPK($t_1$), CT-QPK($t_2$), CT-QPK($t_3$), CT-QPK($t_4$)) and/or the result CT parameter map (V-CT-QPK($t_{max}$)) reflect a distribution of at least one perfusion parameter.

9. Method according to one of the preceding claims, wherein a temporally high-resolution sequence of CT parameter maps (CT-QPK($t_1$), CT-QPK($t_2$), CT-QPK($t_3$), CT-QPK($t_4$), V-CT-QPK($t_{max}$)) is generated on the basis of the determined relation (R) and the MRI image datasets (BD(t)).

10. Method according to claim 9, wherein a monomodal examination, preferably a follow-up examination, is performed on the basis of the temporally high-resolution sequence of CT parameter maps (CT-QPK($t_1$), CT-QPK($t_2$), CT-QPK($t_3$), CT-QPK($t_4$), V-CT-QPK($t_{max}$)) and/or the relation (R).

11. Method for acquiring a quantitative result CT parameter map (V-CT-QPK($t_{max}$)) of a region of interest (ROI) of an object under examination (O), having the following steps:

   - contrast-enhanced acquisition of first raw data (RD1) by spectral multiphase CT of the region of interest (ROI) for at least two predetermined points in time ($t_1$, $t_2$, $t_3$, $t_4$),
   - reconstructing a sequence (SCT) of quantitative input CT parameter maps (CT-QPK($t_1$), CT-QPK($t_2$), CT-QPK($t_3$), CT-QPK($t_4$)) on the basis of the acquired first raw data (RD1) for the at least two predetermined points in time ($t_1$, $t_2$, $t_3$, $t_4$),
   - contrast-enhanced acquisition by means of a 4D MRI measurement of second raw data (RD2) of the region of interest (ROI) with higher temporal resolution compared to multiphase CT,
   - reconstructing, on the basis of the second raw data (RD2), a sequence ($S_{MR}$) of MRI datasets (BD(t)) generated in a contrast-enhanced manner having a higher temporal resolution compared to the sequence ($S_{CT}$) of input CT parameter maps (CT-QPK($t_1$), CT-QPK($t_2$), CT-QPK($t_3$), CT-QPK($t_4$)),
   - performing the method according to one of claims 1 to 10 on the basis of the sequence ($S_{CT}$) of input CT parameter maps (CT-QPK($t_1$), CT-QPK($t_2$), CT-QPK($t_3$), CT-QPK($t_4$)) and the sequence ($S_{MR}$) of MRI image datasets (BD(t)) generated in a contrast-enhanced manner.

12. Parameter determining device (50), having:

- an input interface (51)

- for importing a sequence ($S_{CT}$) of quantitative input CT parameter maps (CT-QPK($t_1$), CT-QPK($t_2$), CT-QPK($t_3$), CT-QPK($t_4$)) of a region of interest (ROI) of an object under examination (O) for at least two predetermined points in time ($t_1$, $t_2$, $t_3$, $t_4$), wherein the sequence ($S_{CT}$) has been generated in a contrast-enhanced manner by spectral multiphase CT
- and for importing a sequence ($S_{MR}$) of MRI image datasets (BD(t)) generated in a contrast-enhanced manner which has a higher temporal resolution compared to the sequence ($S_{CT}$) of input CT parameter maps (CT-QPK($t_1$), CT-QPK($t_2$), CT-QPK($t_3$), CT-QPK($t_4$)),

- a relation determining unit (52) for determining a relation (R) between the MRI image datasets (BD(t)) and the input CT parameter maps (CT-QPK($t_1$), CT-QPK($t_2$), CT-QPK($t_3$), CT-QPK($t_4$))
- a parameter determining unit (53) for determining a result CT parameter map (V-CT-QPK($t_{max}$)) on the basis of the determined relation (R) and the MRI image datasets (BD(t)) for at least one additional point in time ($t_{max}$) which is different from the predetermined points in time ($t_1$, $t_2$, $t_3$, $t_4$).

13. Multimodal medical imaging system (1), having:

- a CT system (2) for acquiring a sequence ($S_{CT}$) of quantitative input CT parameter maps (CT-QPK($t_1$), CT-QPK($t_2$), CT-QPK($t_3$), CT-QPK($t_4$)) of a region of interest (ROI) of an object under examination (O),
- an MRI system (3) for acquiring a sequence ($S_{MR}$) of MRI image datasets (BD(t)) generated in a contrast-enhanced manner of the region of interest (ROI),
- a parameter determining device (50) according to claim 12 for determining a result CT parameter map (V-CT-QPK($t_{max}$)) on the basis of the sequence ($S_{CT}$) of quantitative input CT parameter maps (CT-QPK($t_1$), CT-QPK($t_2$), CT-QPK($t_3$), CT-QPK($t_4$)) and the sequence ($S_{MR}$) of MRI image datasets (BD(t)) generated in a contrast-enhanced manner.

14. Computer program product comprising instructions which, when the program is executed by a computer, cause the computer to carry out the steps of the method according to one of claims 1 to 11.

15. Computer-readable storage medium comprising instructions which, when executed by a computer, cause the computer to carry out the steps of the method according to one of claims 1 to 11.

**Revendications**

1. Procédé de détermination d'une carte (V-CT-QPK($t_{max}$)) de paramètres CT de résultat quantitative dans une partie (ROI) à examiner d'un objet (O) à examiner comportant les stades :

- prise en charge d'une séquence ($S_{CT}$) de cartes (CT-QPK($t_1$), CT-QPK($t_2$)) de paramètres CT d'entrée quantitatives pour au moins deux instants ($t_1$, $t_2$, $t_3$, $t_4$) déterminés à l'avance, dans lequel la séquence ($S_{CT}$) a été créée, avec le soutien d'un agent de contraste, par une CT multiphases spectrale de la partie (ROI) à examiner,
- réception d'une séquence ($S_{MR}$) d'ensembles (BD(t)) de données d'images TRM créée, en étant soutenue par un agent de contraste, qui, par rapport à la séquence ($S_{CT}$) de cartes (CT-QPK($t_1$), CT-QPK($t_2$), CT-QPK($t_3$), CT-QPK($t_4$)) de paramètres CT a une résolution temporelle plus grande,
- détermination d'une relation (R) entre les ensembles (BD(t)) de données d'image TRM et les cartes (CT-QPK($t_1$), CT-QPK($t_2$), CT-QPK($t_3$), CT-QPK($t_4$)) de paramètres CT d'entrée,
- détermination de la carte (V-CT-QPK($t_{max}$)) de paramètres CT de résultat sur la base de la relation (R) déterminée et des ensembles (BD(t)) de données d'image TRM pour au moins un instant ($t_{max}$) supplémentaire, qui se distingue des instants ($t_1$, $t_2$, $t_3$, $t_4$) déterminés à l'avance.

2. Procédé suivant la revendication 1, dans lequel la détermination de la relation (R) comprend les stades :

- association temporelle des ensembles (BD(t)) de données d'image TRM aux cartes (CT-QPK($t_1$), CT-QPK($t_2$), CT-QPK($t_3$), CT-QPK($t_4$)) de paramètres CT d'entrée pour les au moins deux instants ($t_1$, $t_2$, $t_3$, $t_4$) déterminés à l'avance,

- association spatiale relative des cartes (CT-QPK($t_1$), CT-QPK($t_2$)) de paramètres CT d'entrée quantitatives et des ensembles (BD(t)) de données d'image TRM par un enregistrement d'image pour les au moins deux instants ($t_1$, $t_2$, $t_3$, $t_4$) déterminés à l'avance,

- détermination de la relation (R) entre les ensembles (BD(t)) de données d'image TRM 4D associés dans le temps et dans l'espace entre eux et les cartes (CT-QPK($t_1$), CT-QPK($t_2$), CT-QPK($t_3$), CT-QPK($t_4$)) de paramètres CT d'entrée.

3. Procédé suivant la revendication 2, dans lequel la détermination de la relation (R) comprend un rapport entre des premières valeurs (CT($t_1$), CT($t_2$), CT($t_3$), CT($t_4$)) de paramètres CT des cartes (CT-QPK($t_1$), CT-QPK($t_2$), CT-QPK($t_3$), CT-QPK($t_4$)) de paramètres CT d'entrée et des valeurs (SI($t_1$), SI($t_2$), SI($t_3$), SI($t_4$)) d'intensité de signal des ensembles (BD(t)) de données d'image TRM associés dans le temps et dans l'espace aux cartes (CT-QPK($t_1$), CT-QPK($t_2$), CT-QPK($t_3$), CT-QPK($t_4$)) de paramètres CT d'entrée.

4. Procédé suivant la revendication 3, dans lequel la détermination de la carte (V-CT-QPK($t_{max}$)) de paramètres CT de résultat au au moins un instant ($t_{max}$) supplémentaire s'effectue par extrapolation et par interpolation sur la base des premières valeurs (CT($t_1$), CT($t_2$), CT($t_3$), CT($t_4$)) de paramètres CT des cartes (CT-QPK($t_1$), CT-QPK($t_2$), CT-QPK($t_3$), CT-QPK($t_4$)) de paramètres CT d'entrée et sur la base de la relation (R) déterminée.

5. Procédé suivant l'une des revendications précédentes, dans lequel le au moins un instant ($t_{max}$) supplémentaire est déterminé sur la base des ensembles (BD(t)) de données d'image TRM.

6. Procédé suivant l'une des revendications précédentes, dans lequel le au moins un instant ($t_{max}$) supplémentaire comprend un instant ($t_{max}$) où, sur la base des ensembles (BD(t)) de données d'image TRM, on peut s'attendre à une valeur de paramètres CT maximum, de préférence à une valeur (J($t_{max}$)) de concentration d'agent de contraste maximum, dans la partie (ROI) à examiner.

7. Procédé suivant l'une des revendications précédentes, dans lequel les cartes (CT-QPK($t_1$), CT-QPK($t_2$), CT-QPK($t_3$), CT-QPK($t_4$)) de paramètres CT d'entrée et/ou la carte (V-CT-QPK($t_{max}$)) de paramètres CT de résultat indiquent une valeur de paramètre ayant au moins l'un des types de valeurs de paramètre CT suivants :

   - une valeur (CT($t_1$), CT($t_2$), CT($t_3$), CT($t_4$), CT ($t_{max}$)) d'affaiblissement normée,
   - une valeur (HU($t_1$), HU($t_2$), HU($t_3$), HU($t_4$), HU($t_{max}$)) d'affaiblissement dépendant de l'énergie,
   - une valeur de densité,
   - une valeur (J($t_1$), J($t_2$), J($t_3$), J($t_4$), J($t_{max}$)) de concentration de l'agent de contraste.

8. Procédé suivant l'une des revendications précédentes, dans lequel les cartes (CT-QPK($t_1$), CT-QPK($t_2$), CT-QPK($t_3$), CT-QPK($t_4$)) de paramètres CT et/ou la carte (V-CT-QPK($t_{max}$)) de paramètres CT de résultat reproduisent une répartition d'au moins un paramètre de perfusion.

9. Procédé suivant l'une des revendications précédentes, dans lequel, sur la base de la relation (R) déterminée et des ensembles (BD(t)) de données d'image TRM, on produit une séquence à haute résolution dans le temps de cartes (CT-QPK($t_1$), CT-QPK($t_2$), CT-QPK($t_3$), CT-QPK($t_4$), CT-QPK($t_{max}$)) de paramètres CT.

10. Procédé suivant la revendication 9, dans lequel, sur la base de la séquence à haute résolution dans le temps de cartes (CT-QPK($t_1$), CT-QPK($t_2$), CT-QPK($t_3$), CT-QPK($t_4$) , CT-QPK($t_{max}$)) de paramètres CT et/ou de la relation (R), il est produit un examen monomodal, de préférence un examen évolutif.

11. Procédé de saisie d'une carte (V-CT-QPK($t_{max}$)) de paramètres CT de résultat quantitative d'une partie (ROI) à examiner d'un objet (O) à examiner, comportant les stades :

   - acquisition, avec le soutien d'un agent de contraste, de premières données (RD1) brutes par une CT multiphases spectrale de la partie (ROI) à examiner pour au moins deux instants ($t_1$, $t_2$, $t_3$, $t_4$) déterminés à l'avance,
   - reconstruction d'une séquence ($S_{CT}$) de cartes (CT-QPK($t_1$), CT-QPK($t_2$), CT-QPK($t_3$), CT-QPK($t_4$)) de paramètres CT d'entrée quantitatives sur la base des premières données (RD1) brutes acquises pour les au moins deux instants ($t_1$, $t_2$, $t_3$, $t_4$) déterminés à l'avance,

- acquisition, avec le soutien de l'agent de contraste, par une mesure TRM 4D, de deuxièmes données (RD2) brutes de la partie (ROI) à examiner avec une résolution temporelle plus grande que la CT multiphases,
- reconstruction, sur la base des deuxièmes données (RD2) brutes, d'une séquence ($S_{MR}$) d'ensembles (BD(t)) de données d'images TRM créés, avec le soutien d'un agent de contraste, qui a, par rapport à la séquence ($S_{CT}$) de cartes (CT-QPK($t_1$), CT-QPK($t_2$), CT-QPK($t_3$), CT-QPK($t_4$)) de paramètres CT d'entrée, une résolution temporelle plus grande,
- exécution du procédé suivant l'une des revendications 1 à 10, sur la base de la séquence ($S_{CT}$) de cartes (CT-QPK($t_1$), CT-QPK($t_2$), CT-QPK($t_3$), CT-QPK($t_4$)) de paramètres CT d'entrée et la séquence ($S_{MR}$) d'ensembles (BD(t)) de données d'images TRM créés avec le soutien de l'agent de contraste.

12. Dispositif (50) de détermination de paramètres, comportant :

- une interface (51) d'entrée

- pour la prise en charge d'une séquence ($S_{CT}$) de cartes (CT-QPK($t_1$), CT-QPK($t_2$), CT-QPK($t_3$), CT-QPK($t_4$)) de paramètres CT d'entrée quantitatives pour au moins deux instants ($t_1$, $t_2$, $t_3$, $t_4$) déterminés à l'avance, dans lequel la séquence ($S_{CT}$) a été créée, avec le soutien d'un agent de contraste, par une CT multiphases spectrale de la partie (ROI) à examiner de l'objet (O) à examiner,
- et pour la prise en charge d'une séquence ($S_{MR}$) d'ensembles (BD(t)) de données d'images TRM créés, avec le soutien de l'agent de contraste, qui, par rapport à la séquence ($S_{CT}$) de cartes (CT-QPK($t_1$), CT-QPK($t_2$), CT-QPK($t_3$), CT-QPK($t_4$)) de paramètres CT à une résolution temporelle plus grande,

- une unité (52) de détermination de relation pour la détermination d'une relation (R) entre les ensembles (BD(t)) de données d'images TRM et les cartes (CT-QPK($t_1$), CT-QPK($t_2$), CT-QPK($t_3$), CT-QPK($t_4$)) de paramètres CT d'entrée,
- une unité (53) de détermination de paramètres pour la détermination d'une carte (V-CT-QPK($t_{max}$)) de paramètres CT de résultat sur la base de la relation (R) déterminée et des ensembles (BD(t)) de données d'image TRM pour au moins un instant ($t_{max}$) supplémentaire, qui se distingue des instants ($t_1$, $t_2$, $t_3$, $t_4$) déterminés à l'avance.

13. Système (1) d'imagerie médicale multimodale, comportant :

- un système (2) CT d'enregistrement d'une séquence ($S_{CT}$) de cartes (CT-QPK($t_1$), CT-QPK($t_2$), CT-QPK($t_3$), CT-QPK($t_4$)) de paramètres CT d'entrée quantitatives d'une partie (ROI) d'un objet (O) à examiner,
- un système (3) RM d'enregistrement d'une séquence ($S_{MR}$) d'ensembles (BD(t)) de données d'images TRM créés, avec le soutien d'un agent de contraste, de la partie (ROI) à examiner,
- un dispositif (50) de détermination de paramètres suivant la revendication 12, pour la détermination d'une carte (V-CT-QPK($t_{max}$)) de paramètres CT de résultat sur la base de la séquence ($S_{CT}$) de cartes (CT-QPK($t_1$), CT-QPK($t_2$), CT-QPK($t_3$), CT-QPK($t_4$)) de paramètres CT d'entrée quantitatives et de la séquence ($S_{MR}$) des ensembles (BD(t)) de données d'images TRM créés, avec le soutien de l'agent de contraste.

14. Produit de programme d'ordinateur, comprenant des instructions, qui, lors de l'exécution du programme par ordinateur, font qu'elles exécutent les stades du procédé suivant l'une des revendications 1 à 11.

15. Support de mémoire, déchiffrable par ordinateur, comprenant des instructions, qui, lors de l'exécution par l'ordinateur, font que celles-ci exécutent les stades du procédé suivant l'une des revendications 1 à 11.

# FIG 1

100

| 1.I | ROI → RD1 | | 1.III | |
|---|---|---|---|---|

RD1

RD2

| 1.II | | | 1.IV | |
|---|---|---|---|---|

$S_{CT}$

$CT\text{-}QPK(t_1),...,\ CT\text{-}QPK(t_4)$

$S_{MR}$

| 1.V | |
|---|---|

T-BD

| 1.VI | |
|---|---|

R-BD

| 1.VII | T-BD → R |
|---|---|

R

| 1.VIII | $V\text{-}CT\text{-}QPK(t_{max})$ |
|---|---|

## FIG 2

## FIG 3

FIG 4

44

40

42

43

41

FIG 5

50

CT-QPK(t$_1$),..., CT-QPK(t$_4$)

52

51

52a

52b

52c

53

S$_{CT}$

S$_{MR}$

S$_{CT}$

BD(t)

S$_{MR}$

T-BD

R-BD

R

V-CT-QPK(t$_{max}$)

FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **VAN BEERS B. E. et al.** Hepatic Perfusion Parameters in Chronic Disease: Dynamic CT Measurements Correlated with Disease Severity. *AJR,* 2001, vol. 176 (3), 667-687
- **JAHNG G.-H. et al.** Perfusion Magnetic Resonance Imaging: A Comprehensive Update on Principles and Techniques. *Korean Journal of Radiology,* 2014, vol. 15 (5), 554-577
- **ALVAREZ R.E. ; MACOVSKI A.** Energy-selective reconstructions in x-ray computed tomography. *Phys. Med. Biol.,* 1976, vol. 21, 733-744

- **K.L. GRANT et al.** Assessment of an Advanced Image-Based Technique to Calculate Virtual Monoenergetic Computed Tomographie Images From a Dual-Energy Examination to Improve Contrast-To-Noise Ratio in Examinations Using Iodinated Contrast Media. *Investigative Radiology,* 2014, vol. 00, 00-00
- **NADINE SPAHR et al.** Multimodal image registration for liver radioembolization planning and patient assessment. *International Journal of Computer Assisted Radiology and Surgery,* 2019, vol. 14, 215-225